(19) **Europäisches Patentamt / European Patent Office / Office européen des brevets**

(11) **EP 1 643 977 B1**

(12) **EUROPÄISCHE PATENTSCHRIFT**

(45) Veröffentlichungstag und Bekanntmachung des Hinweises auf die Patenterteilung:
**03.10.2007 Patentblatt 2007/40**

(51) Int Cl.:
*A61K 9/26* (2006.01)

(21) Anmeldenummer: **04741056.8**

(22) Anmeldetag: **15.07.2004**

(86) Internationale Anmeldenummer:
**PCT/EP2004/007882**

(87) Internationale Veröffentlichungsnummer:
**WO 2005/007139 (27.01.2005 Gazette 2005/04)**

(54) **MULTIPARTIKULÄRE ARZNEIFORM, ENTHALTEND MUCOADHAESIV FORMULIERTE PEPTID- ODER PROTEIN-WIRKSTOFFE, SOWIE EIN VERFAHREN ZUR HERSTELLUNG DER ARZNEIFORM**

MULTIPARTICLE PHARMACEUTICAL DOSAGE FORM CONTAINING A MUCOADHESIVELY FORMULATED PEPTIDE OR PROTEIN ACTIVE SUBSTANCES METHOD FOR PRODUCING SAID PHARMACEUTICAL DOSAGE FORM

FORME GALENIQUE MULTIPARTICULAIRE CONTENANT DES PRINCIPES ACTIFS PEPTIDIQUES OU PROTEIQUES FORMULES DE FA ON A PRESENTER UNE ACTION MUCOADHESIVE, ET PROCEDE DE PRODUCTION DE CETTE FORME GALENIQUE

(84) Benannte Vertragsstaaten:
**AT BE BG CH CY CZ DE DK EE ES FI FR GB GR HU IE IT LI LU MC NL PL PT RO SE SI SK TR**

(30) Priorität: **15.07.2003 DE 10332160**

(43) Veröffentlichungstag der Anmeldung:
**12.04.2006 Patentblatt 2006/15**

(73) Patentinhaber: **Röhm GmbH**
**64293 Darmstadt (DE)**

(72) Erfinder:
- **LIZIO, Rosario**
  **64380 Rossdorf (DE)**
- **PETEREIT, Hans-Ulrich**
  **64291 Darmstadt (DE)**
- **ROTH, Erna**
  **64297 Darmstadt (DE)**
- **DE ANDRES, Inés**
  **21100 Varese (IT)**
- **DAMM, Michael**
  **63322 Rödermark (DE)**

(56) Entgegenhaltungen:
EP-A- 1 203 590     WO-A-93/13753
WO-A-95/18602     US-B1- 6 468 959

- TRENKTROG TIMM ET AL: "Enteric coated insulin pellets: Development, drug release and in vivo evaluation" EUROPEAN JOURNAL OF PHARMACEUTICAL SCIENCES, Bd. 4, Nr. 6, 1996, Seiten 323-329, XP002314437 ISSN: 0928-0987
- TAKEUCHI H ET AL: "ENTERAL ABSORPTION OF INSULIN IN RATS FROM MUCOADHESIVE CHITOSAN- COATED LIPOSOMES" PHARMACEUTICAL RESEARCH, NEW YORK, NY, US, Bd. 13, Nr. 6, Juni 1996 (1996-06), Seiten 896-901, XP002055070 ISSN: 0724-8741
- DORKOOSH F A ET AL: "Peroral drug delivery systems for peptides and proteins" S.T.P. PHARMA SCIENCES 2002 FRANCE, Bd. 12, Nr. 4, 2002, Seiten 213-221, XP008041739 ISSN: 1157-1489
- BERNKOP-SCHNURCH A: "The use of multifunctional polymers for non-invasive peptide and protein application" EXPERT OPINION ON THERAPEUTIC PATENTS 2000 UNITED KINGDOM, Bd. 10, Nr. 9, 2000, Seiten 1357-1366, XP002314439 ISSN: 1354-3776

**Beschreibung**

Die Erfindung betrifft eine multipartikuläre Arzneiform, enthaltend mucoadhaesiv formulierte Peptid- und/oder Protein-Wirkstoffe, sowie ein Verfahren zur Herstellung der Arzneiform.

**Stand der Technik**

DE 100 24 451 A1 beschreibt pharmazeutische, zur parenteralen Anwendung geeignete Darreichungsformen, die zur Aggregation neigende Peptide in gelöster oder dispergierter Form enthält. Die Peptide können dabei in verschiedenen Salzformen vorliegen. Die Darreichungsformen enthalten zusätzlich freie Säuren und gegebenenfalls weitere pharmazeutische Hilfsstoffe.

WO 02/03955 beschreibt bioadhaesive, microsphaerisch formulierte Arzneiformen zur sublingualen Wirkstoffapplikation. Die Microsphaeren haben einen mittleren Durchmesser von weniger als 50 $\mu$m und enthalten den Wirkstoff, der z. B. ein Peptid sein kann, in nicht kristalliner Form in einer Micromatrix, eingebettet in ein bioadhaesives Polymer. Das bioadhaesive Polymer kann unter anderem eine Cellulose, ein Chitosan oder ein Acrylcopolymer sein.

WO 02/64148 beschreibt Formulierungen enthaltend ein Mucopolysaccharid und ein Verfahren zu ihrer Herstellung. Dabei wird ein Mucopolysaccharid, z. B. Heparin, zusammen mit einem Adsorptionsverstärker, z. B. einem Chitosan, formuliert und anschließend mit einem darmsaftlöslichen Überzug ausgestattet, so daß der Wirkstoff in den mittleren oder unteren Abschnitten des Dünndarms freigesetzt werden kann. Als darmsaftlösliche Überzüge kommen z. B. anionische Acrylcopolymere vom Typ EUPRAGIT® L, S, L100-55 in Betracht. Die Formulierungen können Kapseln, Tabletten und Granulate umfassen.

WO 02/43767 beschreibt orale pharmazeutische Zusammensetzungen für physiologisch aktive Peptid-Wirkstoffe, enthaltend den Wirkstoff, der an einen Zellmembran Überträger gekoppelt ist, ein den pH-Wert absenkendes Mittel und/oder einen Protease Inhibitor sowie ein säurestabiles Transport Vehikel, welches die pharmazeutische Zusammensetzung auf dem Weg durch den Magen des Patienten schützt und einen Kontakt mit den im Magen gegenwärtigen Proteasen verhindert. Bei dem Transport Vehikel kann es sich um Kapseln handeln, die mit säureresistenten Überzügen aus EUDRAGIT® L30 D-55 überzogen sind.

WO 03/007913 beschreibt orale multipartikuläre Arzneiformen, die den Wirkstoff in Form einer Vielzahl sogenannter "Patches" enthalten. Ein "Patch" ist ein diskusartiges Objekt aus biokompatiblen Material mit einem Durchmesser von 500 $\mu$m bis 5 mm und einer Höhe von 100 bis 1000 $\mu$m. Das Patch besteht aus zwei Schichten bzw. Seiten, einer für Wasser oder Körperflüssigkeiten nur gering permeablen Seite, z. B. aus Ethylcellulose, und einer zweiten Seite, die den Wirkstoff, z. B. ein Peptid oder Protein, enthält, der in Mischung mit mucoadhaesiven Polymeren, z. B. Chitosan, CMC, Polyacrylsäure oder Pektin, vorliegen kann. Die Patches können zu einer Tablette verpresst werden oder auch in eine Kapsel gefüllt werden, die zusätzlich mit einem darmsaftlöslichen Überzug ausgestattet wird. Die Wirkstoffpräparationen können auch zusätzlich mit sogenannten Enhancern, wie Fettsäuren, Fettalkoholen, Estern, oberflächenaktiven Substanzen und Proteaseinhibitoren kombiniert werden. Am Wirkort, z. B. im einem bestimmten Darmabschnitt, löst sich die Kapsel auf und gibt die "Patches" frei. Die freigesetzten "Patches" können mit ihrer mucoadhaesiven Seite an der Darmucosa anhaften und dort den Wirkstoff verzögert und auf die Darmucosa gerichtet abgeben. Die zweite, nur gering permeable Seite der "Patches" soll dem Wirkstoff einen gewissen Schutz gegenüber chemischer oder enzymatischer Inaktivierung von der Seite des Darmlumens her bieten und auch verhindern, daß der Wirkstoff nach dieser Seite hin entweicht.

WO 93/13753 beschreibt Peptid-Wirkstoffe enthaltende Pellets mit bioadhäsiver Gelatine-Matrix.

**Aufgabe und Lösung**

Die WO 03/007913 bietet eine beachtliche und anzuerkennende Lösung zur Bereitstellung von oralen Arzneiformen, insbesondere für Wirkstoffe auf Peptid- oder Protein-Basis, die im Darmlumen freigesetzt werden und dort zur Wirkung gelangen sollen. Ein Nachteil dieser Lösung besteht unter anderem in dem aufwendigen Aufbau und der Herstellung der zweischichtigen "Patch"-Strukturen. Ungünstig erscheint insbesondere aber die Bereitstellung der Arzneiform als Kapsel mit einem magensaftresisitenten, darmsaftlöslichen Überzug. Bei einer Größe von deutlich über 2,5 mm ergibt sich hier eine ungenügende therapeutische Reproduzierbarkeit. Die Passagezeit der Kapsel durch den Magen kann stark variieren In jedem Fall ist mit einem verzögerten Wirkungseintritt zu rechnen. Außerdem kann sich die Kapsel schon nach einem teilweisen Auflösen des Überzugs ihrerseits schnell oder langsam auflösen. Beide Prinzipien, Überzug und Kapsel, überlagern sich hier in ungünstiger Weise, so daß mit einer insgesamt unkontrollierten Freisetzung der "Patches" zu rechnen ist. Die Kapsel kann in einem Zustand, wo sie zumindest bereits teilweise den Darmsäften zugänglich ist, je nach aktuellem Darminhalt oder Darmperistaltik intakt bleiben oder auch mechanisch weitgehend zerstört werden. Es kann einerseits zu einer schlagartigen Freisetzung großer Mengen an "Patches" kommen oder andererseits auch zu einer ungewünscht verzögerten Freisetzung, je nach Zerfall oder mechanischer Belastung des zunächst überzogenen Kapselgebildes. Eine insgesamt besser zu kontrollierende Wirkstoffabgabe wäre daher wünschenswert.

Es wurde als eine der Aufgaben der Erfindung gesehen, eine Arzneiform bereitzustellen, die sich zur gezielten und effektiven Freisetzung von Protein- oder Peptidwirkstoffen eignet. Die Arzneiform soll eine hohe Dosiersicherheit bieten und sich nach schneller Magenpassage gut im Darmlumen verteilen. Der enthaltene Protein- oder Peptidwirkstoff soll dabei weitgehend gegenüber physikalischer, chemischer oder proteolytischer Inaktivierung geschützt sein und am definierten Wirkort so freizusetzen sein, daß ein hoher Anteil des Wirkstoffs von Körper aufgenommen werden kann; Der Freisetzungsort soll je nach therapeutischem Ziel variabel und zuverlässig einstellbar sein.

Die Aufgabe wird gelöst durch eine

Orale multipartikuläre Arzneiform, enthaltend Pellets mit einer Größe im Bereich von 50 bis 2500 µm, die aufgebaut sind aus

a) einer inneren Matrix-Schicht, enthaltend einen Wirkstoff, der ein Peptid oder ein Protein einschließlich deren Derivate oder Konjugate ist und in eine Matrix aus einem Polymeren mit mucoadhaesiver Wirkung eingebettet ist, wobei die Matrix optional weitere pharmazeutisch übliche Hilfsstoffe enthalten kann,

b) einem äußeren verfilmten Überzug, bestehend im wesentlichen aus einem anionischen Polymeren oder Copolymeren, das optional mit pharmazeutisch üblichen Hilfsstoffen, insbesondere Weichmachern formuliert sein kann,

dadurch gekennzeichnet, daß

die multipartikuläre Arzneiform so formuliert ist, daß die enthaltenen Pellets im pH-Bereich des Magens freigesetzt werden, der äußere Überzug durch die Wahl des anionischen Polymeren oder Copolymeren bzw. seiner Formulierung mit Hilfsstoffen und seiner Schichtdicke so eingestellt ist, daß sich dieser in pH-Bereichen von 4,0 bis 8,0 im Darm innerhalb 15 bis 60 min auflöst, so daß die wirkstoffhaltige, mucoadhaesive Matrix-Schicht frei wird, an die Darmmucosa binden und dort den Wirkstoff freisetzen kann, wobei das Polymere mit mucoadhaesiver Wirkung so gewählt ist, daß es in einem Bereich +/- 0,5 pH-Einheiten bezogen auf den pH-Wert, bei dem sich der äußere Überzug aufzulösen beginnt, eine mucoadhaesive Wirkung von mindestens $\eta_b$= 150 bis 1000 mPa·s und eine Wasseraufnahme von 10 bis 750 % in 15 min aufweist und der Wirkstoffanteil der Matrixschicht maximal 40 Gew.-% des Gehaltes am Polymeren mit mucoadhaesiver Wirkung beträgt, wobei das Polymere mit mucoadhaesiver Wirkung ein Chitosan, ein (Meth)acrylatcopolymer, bestehend aus 20-40 Gew.-% Methylmethacrylat und 60 bis 80 Gew.-% Methacrylsäure, eine vernetzte und/oder unvernetzte Polyacrylsäure, ein Na-Alginat, und/oder ein Pektin ist.

## Ausführung der Erfindung

Die Erfindung betrifft eine orale multipartikuläre Arzneiform, insbesondere in Form einer Tablette, Minitablette, in Kapseln verfüllter Pellets, Sachets oder Trockensäfte, enthaltend Pellets mit einer mittleren Größe, bzw. mittlerem Durchmesser im Bereich von 50 bis 2500, bevorzugt von 100 bis 1000 µm, die aufgebaut sind aus

a) einer inneren Matrix-Schicht, enthaltend einen Wirkstoff. der ein Peptid oder ein Protein ist einschließlich deren Derivate oder Konjugate und in eine Matrix aus einem Polymeren mit mucoadhaesiver Wirkung eingebettet ist, wobei die Matrix optional bzw. in der Regel weitere pharmazeutisch übliche Hilfsstoffe enthalten kann,

b) einem äußeren verfilmten Überzug, bestehend im wesentlichen aus einem anionischen Polymeren oder Copolymeren, das optional mit pharmazeutisch üblichen Hilfsstoffen, insbesondere Weichmachern formuliert sein kann,

Die multipartikuläre Arzneiform ist so formuliert, daß die enthaltenen Pellets im pH-Bereich des Magens freigesetzt werden.

Der äußere Überzug ist durch die Wahl des anionischen Polymeren bzw. Copolymeren bzw. seiner Formulierung mit Hilfsstoffen und seiner Schichtdicke so eingestellt, daß sich dieser in pH-Bereichen von 4,0 bis 8,0, bevorzugt von 5,5 bis 7,8, besonders bevorzugt 5,8 bis 7,5 im Darm innerhalb 15 bis 60, bevorzugt von 20 bis 40 min auflöst, so daß die wirkstoffhaltige, mucoadhaesive Matrix-Schicht frei wird, an die Darmmucosa binden und dort den Wirkstoff freisetzen kann.

Das Polymere bzw. Copolymere mit mucoadhaesiver Wirkung ist so gewählt, daß es in einem Bereich +/- 0,5, bevorzugt +/- 0,3 pH-Einheiten bezogen auf den pH-Wert, bei dem sich der äußere Überzug aufzulösen beginnt, eine mucoadhaesive Wirkung von mindestens $\eta_b$ = 150 bis 1000, bevorzugt 150 bis 600 mPa·s und eine Wasseraufnahme von 10 bis 750, bevorzugt 10 bis 250, besonders bevorzugt 10 bis 160 % in 15 min aufweist und der Wirkstoffanteil der Matrixschicht maximal 40, insbesondere 0,001 bis 15 oder 0,05 bis 5 Gew.-% des Gehaltes am Polymeren mit mucoadhaesiver Wirkung beträgt.

**Die innere Matrix Schicht**

Die innere Matrix-Schicht fungiert als Wirkstoffträger. Weiterhin hat die innere Matrix-Schicht die Funktion, den Wirkstoff mittels des enthaltenen mucoadhaesiven Polymeren an die Darmmucosa zu binden, so daß dieser von dort aus in den Organismus gelangen kann. Die innere Matrix Schicht hat weiterhin die Funktion den Wirkstoff vor physikalischer, chemischer oder enzymatischer Inaktivierung zu schützen.

**Wirkstoffe/Wirkstoffforrnulierungen**

Die Matrix-Schicht enthält einen Wirkstoff der ein Protein oder Peptid einschließlich deren Derivate oder Konjugate mit einem mittleren Molekulargewicht $M_w$ von 300 bis 1.000.000 (Dalton) sein kann. Unter Derivaten sind chemische oder biochemische Modifikationen der Primär- oder Sekundärstruktur zu verstehen. Beispiele sind aus Naturquellen stammende oder komplett synthetische Proteine oder Peptide, die nicht natürliche Aminosäurereste aufweisen. Konjugate sind kovalente Verknüpfungen von Proteinen oder Peptiden mit nicht peptidischen Verbindungen, z. B. mit Polyethylenglykol gekoppelte Proteine oder Peptide.

Wirkstoffe

Die im Sinne der Erfindung eingesetzten Wirkstoffe sind insbesondere dazu bestimmt, am oder im menschlichen oder tierischen Körper Anwendung zu finden, um

1. Krankheiten, Leiden, Körperschäden oder krankhafte Beschwerden zu heilen, zu lindern, zu verhüten oder zu erkennen.

2. die Beschaffenheit, den Zustand oder die Funktionen des Körpers oder seelische Zustände erkennen lassen.

3. vom menschlichen oder tierischen Körper erzeugte Wirkstoffe oder Körperflüssigkeiten zu ersetzen.

4. Krankheitserreger, Parasiten oder körperfremde Stoffe abzuwehren, zu beseitigen oder unschädlich zu machen oder

5. die Beschaffenheit, den Zustand oder die Funktionen des Körpers oder seelische Zustände zu beeinflussen.

Die Peptid- und Protein-Wirkstoffe können als freie Säuren oder Basen eingesetzt werden. Als Gegenionen können beispielsweise physiologisch Basen oder Säuren, verträgliche Erdalkali- oder Alkalimetalle oder Amine sowie beispielsweise Acetat, Adipat, Ascorbat, Alginat, Benzoat, Benzoolsulfonat, Bromid, Carbonat, Carboxymethylcellulose (freie Säure), Citrat, Chlorid, Dubutylphosphat, Dihydrogencitrat, Dioctylphosphat, Dihexadecylphosphat, Fumarat, Gluconat, Glucuronat, Glutamat, Hydrogencarbonat, Hydrogentartrat, Hydrochlorid, Hydrogencitrat, Jodid, Lactat, alpha-Liponat, Malat, Maleat,Malonat, Pamoat, Palmitat, Phosphat, Salicylat, Stearat, Succinat, sulphat, Tartrat, Tannate, Oleat, Octylphosphat eingesetzt werden.
Der Wirkstoffanteil der Matrixschicht beträgt maximal 40, insbesondere 0,001 bis 15 oder 0,05 bis 5 Gew.-% des Gehaltes am Polymeren mit mucoadhaesiver Wirkung.
In Abhängigkeit der physiko-chemischen Eigenschaften des Wirkstoffs, wie z. B. Partitionskoeffizient Wasser in Öl oder isoselektrischer Punkt etc., kann die Matrix-Schicht zusätzlich eine $C_6$- bis $C_{20}$-, bevorzugt $C_8$-, $C_{10}$-oder $C_{12}$- bis $C_{20}$-Carbon- bzw. Fettsäure und/oder einen $C_6$- bis $C_{20}$-, bevorzugt $C_8$-, $C_{10}$-oder $C_{10}$- bis $C_{20}$-Alkohol einschließlich deren Salze, Ether-, Ester- oder Amid-Derivaten und/oder ein Lipid und/oder ein Phospholipid und/oder ein lipidlösliches Vitamin und/oder einen Proteaseinhibitor und/oder einen Penetrationsförderer und/oder einen Efflux-Pumpen-Inhibitor, z. B. Ketokonazol oder Polyethylen-660-12-Hydroxy-Stearat (Solutol® HS15), enthalten.
Der Wirkstoff kann ein Protein bzw. ein Peptid mit einem mittleren Molekulargewicht $M_w$ von kleiner 3.000 Da sein. Beispiele für solche Peptide sind insbesondere Abarelix, Angiotensin II, Anidulafungin, Antide, Argipressin, Azalin und Azalin B, Bombesin-Antagonist, Bradykinin, Buserelin, Cetrorelix, Ciclosporin A, Desmopressin, Detirelix, Enkephaline (Leu-, Met-) Ganirelix, Gonadorelin, Goserelin, Growthhormone-Secretagogue, Micafungin, Nafarelin, Leuprolide, Leuprorelin, Octreotid, Orntide, Oxytocin, Ramorelix, Sekretin, Somatotropin, Terlipressin, Tetracosactid, Teverelix, Triptorelin, Thyroliberin, Thyrotropin, Vasopressin.
In diesem Fall ist es bevorzugt daß die Matrix-Schicht zusätzlich eine $C_6$- bis $C_{20}$-, bevorzugt $C_8$-, $C_{10}$- oder $C_{12}$- bis $C_{20}$-, gegebenenfalls bis $C_{30}$ Carbon- bzw. Fettsäure und/oder einen $C_6$- bis $C_{20}$-, bevorzugt $C_8$-, $C_{10}$- oder $C_{12}$- bis $C_{20}$-, gegebenenfalls bis $C_{30}$ Alkohol einschließlich deren Salze, Ether-, Ester- oder Amid-Derivaten und/oder ein Lipid und/oder ein Phospholipid und/oder ein lipidlösliches Vitamin und/oder einen Efflux-Pumpen-Inhibitor enthält.

Der Zusatz hat den Vorteil, daß dadurch die Löslichkeit, Stabilität und Aufnahme des Wirkstoffs verbessert werden kann.

Geeignet sind beispielsweise Ester von Fettsäuren wie Glycerintrimyristat, Glycerinmonostearat, Glycerintristearat, Glycerintripalmitat, Glycerylbehensäureester und Fettsäureamide, aliphatische langkettige Carbonsäuren wie Palmitinsäure, Stearinsäure, Laurinsäure, Myristinsäure, Fettalkohole wie Stearylalkohol, Laurylalkohol, , Cetylalkohol, sowie Wachse wie Kanaubawachs, Bienenwachs sowie Phospholipide wie Eilecithin, Sojalecithin, und Vitamine wie Vitamin E.

Der Wirkstoff kann ein Protein oder Peptid mit einem mittleren Molekulargewicht $M_w$ von 3.000 bis 10.000 Da sein. Beispiele für solche Proteine oder Peptide sind insbesondere Calcitonin, Corticotropin, Endorphine, Epithelial growth factor, Glucagon, Insulin, Novolin, Parathyroid hormon, Relaxin, Pro-Somatostatin, Salmon Secretin.

Wenn der Wirkstoff ein Protein oder Peptid mit einem mittleren Molekulargewicht $M_w$ von 3.000 bis 10.000 ist, enthält die Matrix-Schicht bevorzugt eine $C_6$- bis $C_{20}$-, bevorzugt $C_8$-, $C_{10}$- oder $C_{12}$- bis $C_{20}$-, gegebenenfalls bis $C_{30}$ Carbon- bzw. Fettsäure und/oder einen $C_6$- bis $C_{20}$-, bevorzugt $C_8$-, $C_{10}$- oder $C_{12}$- bis $C_{20}$-, gegebenenfalls bis $C_{30}$ Alkohol einschließlich deren Salze, Ether-, Ester- oder Amid-Derivaten und/oder ein Lipid und/oder ein Phospholipid und/oder ein lipidlösliches Vitamin und/oder einen Proteaseinhibitor.

Protein- oder Peptid-Wirkstoffe mit einem mittleren Molekulargewicht $M_w$ von 3.000 bis 10.000 sind oftmals besonders empfindlich gegenüber enzymatischem Abbau durch Proteasen, so daß neben der Stabilisierung des Wirkstoffs an sich der Zusatz von Proteaseinhibitoren von besonderem Vorteil ist.

Pharmazeutisch geeignete Proteaseinhibitoren sind beispielsweise Antipain, Aprotinin, Bacitracin, Benzamidin, Bestatin, Captopril, Chymostatin, Chicken ovoinhibitor, EDTA-Na$_2$, Chitosan-EDTA-conjugates, Na-glycocholate, Leupeptin, Pepstatin, Soybean trypsin inhibitors, Thiorphan, Tos-Lys-Chloromethylketon, Potato carboxypeptidase inhibitor.

Der Wirkstoff kann ein Protein oder Peptid mit einem mittleren Molekulargewicht $M_w$ von mehr als 10.000 sein. Beispiele für solche Proteine oder Peptide sind insbesondere Interferone (alpha, beta, gamma), Interleukine (IL1, IL2), Somatotropin, Erytropoietin, Tumornekrosefaktor (TNF alpha, beta), Relaxin, Endorphin, Dornase alpha, Folikel stimulierendes Hormon (FSH), Human chorion gonadotropin (HCG), Human Growth Hormone Release factor (hGRF), Luteinisierendes Hormon (LH) oder Epidermal Growth Factor.

Wenn der Wirkstoff ein Protein oder Peptid mit einem mittleren Molekulargewicht $M_w$ von mehr als 10.000 Da ist, enthält die Matrix-Schicht bevorzugt zusätzlich eine $C_6$- bis $C_{20}$-, bevorzugt $C_8$-, $C_{10}$- oder $C_{12}$- bis $C_{20}$-, gegebenenfalls bis $C_{30}$ Carbon- bzw. Fettsäure und/oder einen $C_6$- bis $C_{20}$-, bevorzugt $C_8$-, $C_{10}$- oder $C_{12}$- bis $C_{20}$-, gegebenenfalls bis $C_{30}$-Alkohol einschließlich deren Salze, Ether-, Ester- oder Amid-Derivaten und/oder ein Lipid und/oder ein Phospholipid und/oder ein lipidlösliches Vitamin und/oder einen Proteaseinhibitor und/oder einen Penetrationsförderer.

Der Zusatz eines Penetrationsförderers ist vorteilhaft, da die Aufnahme des Wirkstoffs mit vergleichsweise hohem mittleren Molekulargewicht $M_w$ von mehr als 10.000 dadurch begünstigt wird.

Geeignete Penetrationsförderer sind insbesondere Weichmacher wie beispielsweise Triethylcitrat, Acetyltrietylcitrat, Diethylsebacat, Dibutylsebacat, Polymere wie Carbomer, Chitosan, Chitosan-Cystein, NatriumCarboxymethylcellulose, N-Trimethyliertes Chitosan, Polycarbophil-Cysteine, langkettige Fettsäuren, ihre Ester (beispielsweise Mono und Diglyceride) und ihre Salze wie Laurinsäure, Laurinsulfonsäure, Palmitinsäure, Caprylsäure, Caprinsäure, Ölsäure, Acylcarnitine, Chelatbildner wie EDTA, Salicylate, Cyclodextrine, Polyacrylsäuren, Gallensäuren wie Cholsäure, Cholyltaurin, Cholylsarcosin, Chenodeoxycholsäure und ihre Salze wie Na-Cholat, Na-Glykocholat, Na-Taurocholat, Na-Taurodihydrofusidat, Na-Glykodihydrofusidat, Tenside und Emulgatoren wie insbesondere Polyethylen-660-12-Hydroxy-Stearat (Solutol® HS15) (Solutol HS15), Polysorbat 80 (Tween 80), Polyoxyethyliertes Kastoröl (Cremophor EL), Polyoxyethylene-polyoxypropylene glycol (Pluronic® F68), das Toxin Zonula Occludens Toxin (ZOT) sowie Vitamine wie Vitamin E (Tocopherol) oder Vitamin B12.

Wenn der Wirkstoff ein Protein oder Peptid mit einem hohen Molekulargewicht $M_w$ von mehr als 10.000 ist, enthält die Matrix-Schicht zusätzlich bevorzugt einen Effluxpumpen-Inhibitor wie insbesondere Ketokonazol oder Polyethylen-660-12-Hydroxy-Stearat (Solutol HS15).

Polymere mit mucoadhaesiver Wirkung

Die Matrix-Schicht enthält weiterhin Polymere mit mucoadhaesiver Wirkung, wobei das Polymere mit mucoadhaesiver Wirkung ein Chitosan, ein (Meth)acrylatcopolymer, bestehend aus 20-40 Gew.-% Methylmethacrylat und 60 bis 80 Gew.-% Methacrylsäure, eine vernetzte und/oder unvernetzte Polyacrylsäure, ein Na-Alginat, und/oder ein Pektin ist.

Das Polymer mit mucoadhaesiver Wirkung ist so gewählt, daß es in einem Bereich +/- 0,5, bevorzugt +/- 0,3 pH-Einheiten bezogen auf den pH-Wert, bei dem sich der äußere Überzug aufzulösen beginnt, eine Wasseraufnahme von 10 bis 750, bevorzugt 10 bis 250, besonders bevorzugt 10 bis 160 % in 15 min aufweist.

## Messung der mucoadhäsiven Eigenschaften

Eine geeignete Messmethode zur Charakterisierung mucoadhäsiver Eigenschaften ist in *Hassan und Gallo* (1990) enthalten (s. Hassan E.E. und Gallo J. M. "A Simple Rheological Method for the in Vitro Assessment of Mucin-Polymer Bioadhaesive Bond Strength" Pharma Res. 7 (5), 491 (1990)). Die Methode beruht auf der Annahme, daß die Viscosität ($\eta$, dynamische Viscosität bzw. Viscositätskoeffizient) einer Mischung von Polymeren mit Mucin verschieden ist, von der Summe der Viskositäten der Einzelkomponenten. Es gilt der Zusammenhang

$$\eta_{\text{Mischung Polymer mit Mucin}} = \eta_{\text{Mucin}} + \eta_{\text{Polymer}} + \eta_b \, ,$$

wobei $\eta_b$ für die Differenz steht. Je höher $\eta b$ , desto höher sind die mucoadhaesiven Eigenschaften. Die einzelnen Komponenten werden zunächst mit einem Rotationsviscosimeter auf ihre Viscosität hin vermessen. Eingesetzt werden eine 0,5 %-ige (w/w) wässrige Lösung des mucoadhaesiven Polymeren und eine 15 %-ige Lösung von Mucin aus Schweinemagen. Zur Bestimmung der mucoadhaesiven Eigenschaften $\eta_b$ werden Mucin und Polymer allein und in ihrer Mischung in den angegebenen Konzentrationen gemessen.

Das Polymer mit mucoadhaesiver Wirkung ist so gewählt, daß es in einem Bereich +/- 0,5, bevorzugt +/- 0,3 pH-Einheiten bezogen auf den pH-Wert, bei dem sich der äußere Überzug aufzulösen beginnt, eine mucoadhaesive Wirkung gemessen als Viskosität $\eta_b$ von 150 bis 1000, bevorzugt 150 bis 600, mPa·s aufweist.

## Hydratisierung und Wasseraufnahme

Die Hydratisierung von Polymeren beruht auf der Affinität des Polymers Wasser aufzunehmen. Polymere schwellen durch diese Wasseraufnahme an. Dies liegt an einem Ungleichgewicht zwischen dem chemischen Potential des Wassers im Polymer und dem Wasser im umgebenden Medium. Das Wasser wird aufgrund des osmotischen Druckes des Polymers solange aufgenommen bis sich ein Gleichgewicht zwischen innerer und äußerer Phase eingestellt hat. Das Polymer ist dann zu 100% hydratisiert. Für Polymere mit niedrigem mittleren Molekulargewicht liegt dann eine Lösung vor. Für Polymere mit höherem Molekulargewicht oder vernetzten Polymeren entsteht ein Gel. Die Wasseraufnahme bis zur Gleichgewichtseinstellung kann z. B. bis zum 10 fachen des eigenen Gewichtes betragen entsprechend 1000% des Polymergewichtes.

## Messung der prozentualen Wasseraufnahme

Die Messung der prozentualen Wasseraufnahme ist dem Fachmann geläufig. Eine geeignete Methode ist z. B. im Lehrbuch der pharmazeutischen Technologie/Rudolf Voigt, Basel: Verlag Chemie, 5. völlig überarbeitete Auflage, 1984, S. 151, 7.7.6 unter "Aufsaugvermögen" beschrieben. Die Methode bedient sich der sogenannten Enslin-Apparatur, bei der eine Glasfilternutsche über einen Schlauch mit einer graduierte Pipette verbunden ist. Die Pipette ist genau horizontal so montiert, daß sie auf gleicher Höhe mit der Glasfritte liegt. Eine Wasseraufnahme von 100 % wird im vorliegenden Fall als eine Wasseraufnahme von 1 ml Wasser pro 1 g Polymeren mit mucoadhaesiver Wirkung in 15 min definiert.

Durch die vergleichsweise schnelle Wasseraufnahme bzw. Hydratisierung und den hohen Hydratisierungsgrad wird zu dem Zeitpunkt, ab dem sich der äußere Überzug aufzulösen beginnt, ein rascher Schutz des Wirkstoffs und eine unmittelbare Bindung an die Darmmucosa gewährleistet. Die Bindung des Wirkstoffs in der mucoadhaesiven Matrix soll nur gering sein, so daß dieser unmittelbar von der Darmmucosa in den Organismus übergehen kann.

## Steuerung des Matrix-pH-Wertes

Die mucoadhaesive Wirkung ist bei vielen mucoadhaesiven Polymeren pHabhängig. Der pH-Wert in der Matrix kann gezielt durch den Zusatz einer Säure, einer Base oder eines Puffersystems gesteuert werden. Die innere Matrix kann beispielsweise als Polymer mit mucoadhaesiver Wirkung ein Chitosan enthalten, das zusammen mit einem Acetat-Puffersystem eingesetzt wird. Der Acetat/Na-Acetat-Puffer, z. B. auf pH 5,0 bis 5,5 eingestellt, kann sich als Zusatzstoff in der Matrix befinden oder auf einem Kern auf dem die Matrix aufgetragen wird aufgebracht. Auf diese Weise kann Chitosan auch in Kombination mit verfilmten Überzügen eingesetzt werden, die sich bei höheren pH-Werten, z. B. pH 6,0 bis 8,0, aufzulösen beginnen. Trotz des hohen Umgebungs-pH-Wertes, bleibt der niedrige pH-Wert in der Mikroumgebung der Matrix erhalten. Man kann so die mucoadhaesiven Eigenschaften des Polymers in einem pH-Bereich nutzen, in dem es ansonsten nicht oder nicht in diesem Maße mucoadhaesiv wirken würde. Dies hat den Vorteil, daß man einen gewissen Schutz gegenüber Proteasen erzielen kann, deren pH-Optimum in höheren pH-Bereichen liegt. Das gleiche Prinzip kann auch in umgekehrter Weise angewendet werden, indem man den pH-Wert der Matrix mittels des Zusatzes einer Base erhöht und mit einem verfilmten Überzug kombiniert, der sich bei niedrigeren pH-Werten auflöst.

Beispiele zur Auswahl geeigneter mucoadhaesiver Polymere

Die Auswahl geeigneter mucoadhaesiver Polymere basiert auf ihren mucoadhaesiven Eigenschaften und ihrer Wasseraufnahmefähigkeit. Die Polymere sollen im jeweiligen pH-Bereich eine mucoadhaesive Wirkung von mindestens $\eta_b$= 150 bis 1000 mPa·s und eine Wasseraufnahme von 10 bis 750 % in 15 min aufweisen. Die folgende Tabelle gibt eine beispielhafte Auflistung.

Chitosan ist z. B. geeignet für die Anwendung in einem Umgebungs-pH-Bereich von pH 5,5 (Duodenum) oder bei einem anderen Umgebungs-pH-Bereich (Ileum oder Colon), sofern der Matrix-pH-Bereich, z. B. mit Hilfe eines Puffersystems, auf den Bereich um pH 5,5 eingestellt wurde.

Das in der Tabelle aufgelistete (Meth)acrylatcopolymer ist besser für einen Ph-Bereich von pH 7,2 als für einen pH-Berich um pH 5,5 geeignet.

Na-Alginat ist für den pH-Bereich um pH 5,5 geeignet, jedoch nicht für pH 7,2.

Na-Carboxymethylcellulose und vernetzte Polyacrylsäure sind über einen weiten pH-Bereich von 5,5 bis 7,2 geeignet.

| Mucoadhesives Polymer | Mucoadhaesive Wirkung $\eta_b$ [mPa·s] bei pH 5,5 | Mucoadhaesive Wirkung $\eta_b$ [mPa·s] bei pH 7,2 * | $H_2O$-Aufnahme [% in 15 min] bei pH 5,5 | $H_2O$-Aufnahme [% in 15 min] bei pH 6,0 | $H_2O$-Aufnahme [% in 15 min] bei pH 7,2 |
|---|---|---|---|---|---|
| Chitosan | 220 | 0 | 140 | 320 | 320 |
| (Meth) acrylatcopolymer* | 150 | 480 | 170 | 50 | 125 |
| Na-Alginat | 580 | 0 | 40 | 50 | 50 |
| Na-Carboxymethylcellulose | 300 | 250 | 55 | 50 | 50 |
| Polyacrylsäure vernetzt | 350 | 340 | 50 | 25 | 25 |
| *= (Meth)acrylatcopolymer aus 30 Gew.-% Methylmethacrylat und 70 Gew.-% Methacrylsäure | | | | | |

## Der äußere Überzug aus anionischen (Meth)acrylatcopolymeren

Der äußere Überzug aus anionischen Polymeren oder Copolymeren dient als magensaftresistenter Überzug zum Schutz der inneren Matrixschicht vor den Magensäften. Weiterhin fungiert der äußere Überzug zum Schutz des Wirkstoffs vor proteolytischen Enzymen bis zu dem Zeitpunkt, an dem sich der Überzug einen Darmabschnitt (Duodenum, Jejunum, Ileum oder Colon) erreicht, an dem er sich aufzulösen beginnt. Der äußere Überzug dient dabei insbesondere dem sogenannten gastrointestinalen Targeting", d. h. der gezielten Freisetzung der inneren Matrixschicht, an den durch ihren dort herrschenden pH-Wert bestimmten Darmabschnitten. Damit es nicht zu einer Behinderung der Abgabe der inneren Matrix-Schicht kommt, soll das (Meth)acrylatcopolymere des äußeren Überzugs möglichst keine oder nur geringe Wechselwirkungen mit dem Wirkstoff oder dem mucoadhaesiven Polymeren der inneren Matrixschicht aufweisen.

Geeignete anionische Polymere bzw. Copolymere sind Celluloseglycolat (Duodcell®), Celluloseacetatphtalat (CAP, Cellulosi acetas, PhEur, Celluloseacetate-phtalate, NF, Aquaterio®), Celluloseacetatsuccinat (CAS), Celluloseacetattrimeliat (CAT), Hydroxypropylmethylcellulosephtalat (HPMCP, HP50, HP55), Hydroxypropylmethylcelluloseacetatsuccinat (HPMCAS -LF, -MF, -HF), Polyvinylacetatphtalat (PVAP, Sureteric®), Vinylacetat-Vinylpyrolidon-Copolymer (PVAc, Kollidon® VA64), Vinylacetat:Crotonsäure-Copolymer 9:1 (VAC:CRA, Kollicoat® VAC) und oder Shelllack. Die genannten Polymere bzw. Copolymere lassen sich vielfach in durchaus befriedigender Weise so formulieren, daß eine pH-spezifische Auflösung erreicht werden kann.

Besonders bevorzugt besteht der äußere verfilmte Überzug im wesentlichen aus (Meth)acrylat-Copolymeren mit einem Gehalt an Monomeren mit anionischen Gruppen von 5 bis 60 Gew.-%, die optional mit pharmazeutisch üblichen Hilfsstoffen, insbesondere Weichmachern formuliert sein können. Gegenüber den eingangs genannten Polymeren bieten die genannten anionischen (Meth)acrylatcopolymere im Rahmen der Erfindung die Möglichkeit in vielen Fällen eine noch genauere und reproduzierbarere pH-spezifische Einstellung des Auflöse-pH-wertes einzustellen. Auch die Handhabung und Applikation wird in der Regel als weniger aufwendig angesehen.

Das (Meth)acrylat-Copolymere für den äußeren Überzug besteht bevorzugt zu 40 bis 95, bevorzugt zu 45 bis 90, insbesondere zu 30 bis Gew.-% aus radikalisch polymerisierten $C_1$- bis $C_4$-Alkylestern der Acryl- oder der Methacrylsäure und kann 5 bis 60, bevorzugt 8 bis 40, insbesondere 20 bis 35 Gew.-% (Meth)acrylat-Monomere mit einer anionischen Gruppe enthalten.

In der Regel addieren sich die genannten Anteile zu 100 Gew.-%. Es können jedoch zusätzlich, ohne daß dies zu einer Beeinträchtigung oder Veränderung der wesentlichen Eigenschaften führt, geringe Mengen im Bereich von 0 bis 10, z. B. 1 bis 5 Gew.-% weiterer vinylisch copolymerisierbarer Monomere, wie z. B. Hydroxyethylmethacrylat oder Hydroxyethylacrylat enthalten sein.

$C_1$- bis $C_4$-Alkylestern der Acryl- oder Methacrylsäure sind insbesondere Methylmethacrylat, Ethylmethacrylat, Butylmethacrylat, Methylacrylat, Ethylacrylat und Butylacrylat.

Ein (Meth)acrylat-Monomer mit einer anionischen Gruppe kann z. B. Acrylsäure, bevorzugt jedoch Methacrylsäure sein.

Weiterhin geeignet sind anionische (Meth)acrylat Copolymere aus 40 bis 60, Gew.-% Methacrylsäure und 60 bis 40 Gew.-% Methylmethacrylat oder 60 bis 40 Gew.-% Ethylacrylat (Typen EUDRAGIT® L oder EUDRAGIT® L100-55).

EUDRAGIT® L ist ein Copolymer aus 50 Gew.-% Methylmethacrylat und 50 Gew.-% Methacrylsäure. EUDRAGIT® L 30D ist eine Dispersion enthaltend 30 Gew.-% EUDRAGIT® L. Dieses (Meth)acrylatcopolymer ist besonders geeignet zur Auflösung in pH-Bereichen um pH 6,0 bis 6,5 (Jejunum).

EUDRAGIT® L100-55 ist ein Copolymer aus 50 Gew.-% Ethylacrylat und 50 Gew.-% Methacrylsäure. EUDRAGIT® L 30-55 ist eine Dispersion enthaltend 30 Gew.-% EUDRAGIT® L 100-55. Dieses (Meth)acrylatcopolymer ist besonders geeignet zur Auflösung in pH-Bereichen um pH 5,5 bis 6,0 (Duodenum)

Ebenso geeignet sind anionische (Meth)acrylat Copolymere aus 20 bis 40 Gew.-% Methacrylsäure und 80 bis 60 Gew.-% Methylmethacrylat (Typ EUDRAGIT® S). Dieses (Meth)acrylatcopolymer ist besonders geeignet zur Auflösung in pH-Bereichen um pH 6,5 bis 7,0 (Jejunum bzw. Ileum) Besonders gut geeignet sind (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure.

EUDRAGIT® FS ist ein Copolymer aus 25 Gew.-%, Methylmethacrylat, 65 Gew.-% Methylacrylat und 10 Gew.-% Methacrylsäure. EUDRAGIT® FS 30 D ist eine Dispersion enthaltend 30 Gew.-% EUDRAGIT® FS. Dieses (Meth)acrylatcopolymer ist besonders geeignet zur Auflösung in pH-Bereichen um pH 7,0 bis 7,8 (Ileum bzw. Colon)

Weiterhin geeignet ist ein Copolymer, welches sich aus

20 bis 34 Gew.-% Methacrylsäure und/oder Acrylsäure,
20 bis 69 Gew.-% Methylacrylat und
0 bis 40 Gew.-% Ethylacrylat und/oder gegebenenfalls
0 bis 10 Gew.-% weiteren vinylisch copolymerisierbarer Monomeren

zusammensetzt, mit der Maßgabe, daß die Glastemperatur des Copolymers nach ISO 11357-2, Punkt 3.3.3, höchstens 60 °C beträgt. Dieses (Meth)acrylatcopolymer ist wegen seiner guten Reißdehungseigenschaften insbesondere zum Verpressen von Pellets zu Tabletten geeignet.

Weiterhin geeignet sind Copolymere aus

20 bis 33 Gew.-% Methacrylsäure und/oder Acrylsäure,
5 bis 30 Gew.-% Methylacrylat und
20 bis 40 Gew.-% Ethylacrylat und
größer 10 bis 30 Gew.-% Butylmethacrylat und
gegebenenfalls
0 bis 10 Gew.-% weiteren vinylisch copolymerisierbarer Monomeren,
wobei sich die Anteile der Monomeren zu 100 Gew.-% addieren,

zusammensetzt, mit der Maßgabe, daß die Glastemperatur des Copolymers (glass transition temperature) nach ISO 11357-2, Punkt 3.3.3 (midpoint temperature $T_{mg}$), 55 bis 70 °C beträgt. Copolymere dieses Typs sind wegen seiner guten mechanischen Eigenschaften insbesondere zum Verpressen von Pellets zu Tabletten geeignet.

Das oben genannte Copolymer setzt sich insbesondere zusammen aus radikalisch polymerisierten Einheiten von

20 bis 33, bevorzugt 25 bis 32, besonders bevorzugt 28 bis 31 Gew.-% Methacrylsäure oder Acrylsäure, bevorzugt ist Methacrylsäure,

5 bis 30, bevorzugt 10 bis 28, besonders bevorzugt 15 bis 25 Gew.-% Methylacrylat,

20 bis 40, bevorzugt 25 bis 35, besonders bevorzugt 18 bis 22 Gew.-% Ethylacrylat, sowie

größer 10 bis 30, bevorzugt 15 bis 25, besonders bevorzugt 18 bis 22 Gew.-% Butylmethacrylat

zusammen, wobei die Monomerzusammensetzung so gewählt wird, daß die Glastemperatur des Copolymers 55 bis 70 °C, bevorzugt 59 bis 66, besonders bevorzugt 60 bis 65 °C beträgt.

Zur Einstellung spezieller Freisetzungsprofile bzw. Freisetzungsorte können auch Mischungen der genannten Copolymere zum Einsatz kommen.

Unter Glastemperatur wird hier insbesondere die midpoint temperature $T_{mg}$ nach ISO 11357-2, Punkt 3.3.3, verstanden. Die Messung erfolgt ohne Weichmacherzusatz, bei Restmonomergehalten (REMO) von weniger als 100 ppm, bei einer Aufheizrate von 10 °C/min und unter Stickstoffatmosphäre.

Das Copolymer besteht bevorzugt in wesentlichen bis ausschließlich, zu 90, 95 oder 99 bis 100 Gew.-%, aus den Monomeren Methacrylsäure, Methylacrylat, Ethylacrylat und Butylmethacrylat in den oben angegebenen Mengenbereichen.

Es können jedoch zusätzlich, ohne daß dies zu einer Beeinträchtigung der wesentlichen Eigenschaften führen muß, geringe Mengen im Bereich von 0 bis 10, z. B. 1 bis 5 Gew.-% weiterer vinylisch copolymerisierbarer Monomere, wie z. B. Methylmethacrylat, Butylacrylat, Hydroxyethylmethacrylat, Vinylpyrrolidon, Vinylmalonsäure, Styrol, Vinylalkohol, Vinylacetat und/oder deren Derivate enthalten sein.

Die Copolymere werden in an sich bekannter Weise durch radikalische Substanz-, Lösungs-, Perl- oder Emulsionspolymerisation erhalten. Sie müssen vor der Verarbeitung durch geeignete Mahl-, Trocken- oder Sprühprozesse in den erfindungsgemäßen Teilchengrößenbereich gebracht werden. Dies kann durch einfaches Brechen extrudierter und abgekühlter Granulatstränge oder Heißabschlag erfolgen.

Insbesondere bei Mischung mit weiteren Pulvern oder Flüssigkeiten kann der Einsatz von Pulvern vorteilhaft sein. Geeignete Gerätschaften zur Herstellung der Pulver sind dem Fachmann geläufig, z. B. Luftstrahlmühlen, Stiftmühlen, Fächermühlen. Gegebenenfalls können entsprechende Siebungsschritte einbezogen werden. Eine geeignete Mühle für industrielle Großmengen ist zum Beispiel eine Gegenstrahlmühle (Multi Nr. 4200), die mit ca. 6 bar Überdruck betrieben wird.

### Copolymerherstellung

Die genannten (Meth)acrylatcopolymere sind durch radikalische Polymerisation der Monomeren erhältlich (siehe z. B. EP 0 704 207 A2 und EP 0 704 208 A2). Die Copolymere sind in an sich bekannter Weise durch radikalische Emulsionspolymerisation in wäßriger Phase in Gegenwart von vorzugsweise anionischen Emulgatoren herstellbar, beispielsweise nach dem in DE-C 2 135 073 beschriebenen Verfahren.

### Organische Lösung

Die genannten (Meth)acrylatcopolymere können in Form einer organischen Lösung, z. B. in einer Konzentration von 10 bis 30 Gew.-%, bereitgestellt werden. Als Lösungsmittel können z. B. Aceton, Isopropanol oder Ethanol oder Mischung daraus verwendet werden, die gegebenenfalls Wasseranteile bis etwa 10 Gew.-% enthalten können. Bevorzugt sind jedoch wäßrige Dispersionen.

### Dispersionen

Die genannten (Meth)acrylatcopolymere können als Emulsionspolymerisate vorzugsweise in Form einer 10- bis 50-gew.-prozentigen, insbesondere 20 bis 40-prozentigen wäßrigen Dispersion erzeugt und angewendet werden. Als Handelsform ist ein Feststoffgehalt von 30 Gew.-% bevorzugt. Für die Verarbeitung ist eine teilweise Neutralisation der Methacrylsäure-Einheiten entbehrlich; sie ist jedoch, beispielsweise in einem Umfang bis zu 5 oder 10 Mol-% möglich, wenn eine Stabilisierung oder Verdickung der Überzugsmitteldispersion erwünscht sein sollte. Der Gewichtsmittelwert der Latex-Teilchengröße beträgt in der Regel 40 bis 100 nm, vorzugsweise 50 bis 70 nm, was eine verarbeitungstechnisch günstige Viskosität unter 1000 mPa · s gewährleistet.

Bei höheren Neutralisationsgrades z. B. 10 bis 50 Mol.-% oder vollständiger Neutralisation ist es möglich, das Copolymer in einen gelösten Zustand zu überführen.

Um eine Lösung aes anionischen Copolymers herzustellen ist in der Regel eine teilweise oder vollständige Neutralisation der Säuregruppen nötig. Das anionische Copolymer kann z. B. nach und nach in einer Endkonzentration von 1 bis 40 Gew.-% in Wasser eingerührt werden und dabei teilweise oder vollständig neutralisiert werden durch Zugabe einer basischen Substanz wie z. B. NaOH, KOH, Ammoniumhydroxyd oder organische Basen wie z. B. Triethanolamin. Es ist auch möglich ein Pulver des Copolymeren einzusetzen, dem bereits bei seiner Herstellung zum Zweck der (Teil) neutralisation eine Base z. B. NaOH zugesetzt wurde, so daß das Pulver ein bereits (teil)neutralisiertes Polymer ist. Der pH-Wert der Lösung liegt in der Regel über 4, z. B. im Bereich von 4 bis ca. 7.

Die Dispersion kann z. B. auch in an sich bekannter Weise sprühgetrocknet oder gefriergetrocknet werden und im Form eines redispergierbaren Pulvers bereitgestellt werden (siehe z. B. EP-A 0 262 326). Alternative Verfahren sind die Gefriertrocknung oder Coagulation uns Abquetschen des Wassers in einem Extruder mit anschließender Granulation (siehe z. B. EP-A 0 683 028).

Überraschenderweise wurde gefunden, daß Copolymer-Dispersionen aus sprüh- oder gefriergetrockneten und redispergierten Pulvern eine erhöhte Scherstabilität aufweisen. Dies ist insbesondere beim Sprühauftrag von Vorteil. Dieser Vorteil tritt insbesondere verstärkt hervor wenn das in der Dispersion enthaltene Copolymer zu 2 bis 10 Mol-% in teilneutralisierter Form vorliegt (bezogen auf die im Copolymer enthaltenen Säuregruppen). Bevorzugt ist zu diesem Zweck die Teilneutalisation mittels Zugabe von NaOH. Bevorzugt ist ein anionischer Emulgator in Menge von 0,1 bis 2 Gew.-% enthalten. Besonders bevorzugt ist Natiumlaurylsulfat als Emulgator.

Schichtdicken

Die Schichtdicke des äußeren Überzuges liegt bevorzugt im Bereich von 20 bis 200, bevorzugt von 50 bis 120 μm.

**Herstellung einer multipartikulären Arzneiform**

Die Erfindung betrifft weiterhin ein Verfahren zur Herstellung einer multipartikulären Arzneiform, indem man

a) eine innere Matrix-Schicht, enthaltend einen Wirkstoff, der ein Peptid oder ein Protein ist und ein Polymer mit mucoadhaesiver Wirkung und gegebenenfalls weitere pharmazeutisch üblichen Hilfsstoffe mittels Sprühauftrag auf einen Kern oder durch Rotagglomeration, Ausfällen oder Sprühverfahren ohne einen Kern herstellt und anschließend b) einen äußeren verfilmten Überzug. bestehend im wesentlichen aus einem anionischen Polymeren, das optional mit pharmazeutisch üblichen Hilfsstoffen, insbesondere Weichmachern, formuliert sein kann, mittels Sprühauftag aufbringt, so daß wirkstoffhaltige, umhüllte Pellets erhalten werden, und man c) die erhaltenen Pellets mittels pharmazeutisch üblicher Hilfsstoffe und in an sich bekannter Weise zu einer multipartikulären Arzneiform, insbesondere zu pellethaltigen Tabletten, Minitabletten, Kapseln, Sachets oder Trockensäften verarbeitet, die so formuliert sind, daß die enthaltenen Pellets im pH-Bereich des Magens freigesetzt werden.

Herstellung von Prä-Pellets und Pellets

Die Pelletierung kann auf wirkstofffreie Kugeln (Nonpareilles) erfolgen oder es können kernfreie Pellets erzeugt werden.

Zunächst wird die innere Matrixschicht mit oder ohne Kern erzeugt. Man diese noch nicht überzogene, ausgerundete Schicht als Prä-Pellet (Pelletkern) bezeichnen.

Mittels eines Wirbelschichtverfahrens kann die Flüssigkeit auf Placebopellets oder sonstige geeignete Trägermaterialien aufgebracht werden, wobei das Lösungs- oder Suspendiermittel verdunstet wird. Nach dem Herstellverfahren kann sich ein Trocknungsschritt anschließen.

Der Peptid- oder Proteinwirkstoff wird mit dem Polymeren mit mucoadhaesiver Wirkung in einem organischen Lösemittel oder in Wasser eingetragen und vermischt. Um eine befriedigende Sprühbarkeit des Gemisches zu gewährleisten, ist es meist notwendig, ein Gemisch mit niedriger Viscosität zu formulieren. Zu diesem Zweck kann es günstig sein, das Polymere mit mucoadhaesiver Wirkung in vergleichsweise niedrigen Konzentrationen, z. B. von 1 bis höchstens 10, bevorzugt 2 bis 5 Gew.-% einzusetzen. Weiterhin kann der Zusatz eines Detergenz, z. B. Tween in Konzentrationen von 0,1 bis 20, bevorzugt 0,5 bis 10 Gew.-% zur Herabsetzung der Oberflächenspannung vorteilhaft sein.

Neben dem Wirkstoff können sie weitere pharmazeutische Hilfsstoffe enthalten: Bindemittel, wie Zellulose und deren Derivate, Polyvinylpyrrolidon (PVP), Feuchthaltemittel, Zerfallsförderer, Gleitmittel, Sprengmittel, (Meth)acrylate, Stärke und deren Derivate, Zucker Solubilisatoren oder andere.

Entsprechende Auftragsverfahren sind z. B. aus Bauer, Lehmann, Osterwald, Rothgang, "Überzogene Arzneiformen" Wissenschaftliche Verlagsgesellschaft mbH Stuttgart, Kap 7, S. 165 - 196, bekannt.

Details sind dem Fachmann weiterhin aus Lehrbüchern bekannt. Siehe z. B.:

- Voigt, R. (1984): Lehrbuch der pharmazeutischen Technologie; Verlag Chemie Weinheim - Beerfield Beach/Florida - Basel.
- Sucker, H., Fuchs, P., Speiser, P. : Pharmazeutische Technologie, Georg Thieme Verlag Stuttgart (1991), insbesondere Kapitel 15 und 16, S. 626 - 642.
- Gennaro, A.,R. (Editor), Remington's Pharmaceutical Sciences, Mack Publishing Co., Easton Pennsylvania (1985), Chapter 88, S. 1567 - 1573.
- List, P. H. (1982): Arzneiformenlehre, Wissenschaftliche Verlagsgesellschaft mbH, Stuttgart.

Die innere Matrix kann auch ohne Zuhilfenahme eines inerten Kerns (Nonpareilles) erzeugt werden. Die Inhaltsstoffe der inneren Matrix können dabei durch Verfahren wie Rotagglomeration, Ausfällen oder Sprühverfahren, insbesondere Ultraschall-Wirbel-Sprühverfahren, zu noch nicht überzogenen Pellets (Prä-Pellets) definierter Größe, z. B. 50 bis 1000 μm, ausgerundet werden. Dies hat den Vorteil, daß das gesamte Kernvolumen zur Wirkstoffbeladung zur Verfügung steht. Die Wirkstoffbeladung kann dadurch gegenüber der Ausführungsform mit inertem Kern nochmals erhöht werden.

Nach Herstellung der inneren Matrix-Kerne (bzw. der Prä-Pellets) werden diese wiederum bevorzugt im Sprühverfahren mit dem äußeren Überzug versehen, so daß man fertige Pellets erhält. Die Herstellung der Pellets erfolgt mittels Sprühauftrag aus organischer Lösung, oder bevorzugt aus wäßrigen Dispersionen. Für die Ausführung ist dabei entscheidend, daß gleichmäßige, porenfreie Überzüge entstehen.

Topcoat

Die Pellets können zusätzlich mit pigmentierten Überzügen versehen werden, die jedoch nicht den Auflöse-pH-Wert beeinflussen dürfen. Geeignet sind z. B. Überzüge aus pigmentierter Hydroxypropylmethylcellulose oder anderen wasserlöslichen bzw. in Wasser schnell zerfallenden Polymeren.

**Pharmazeutisch übliche Hilfsstoffe**

Den erfindungsgemäßen Formulierungen können bei der Herstellung übliche Hilfs- bzw. Zuschlagstoffe hinzugefügt werden. Grundsätzlich müssen natürlich alle eingesetzten Substanzen toxikologisch unbedenklich und insbesondere in Arzneimitteln ohne Risiko für Patienten zu verwenden sein.

Einsatzmengen und Verwendung der üblichen Zuschlagstoffe in Arzneimittelüberzügen oder Beschichtungen sind dem Fachmann geläufig. Übliche Zuschlagstoffe können z. B. Weichmacher, Trennmittel, Pigmente, Stabilisatoren, Antioxidantien, Porenbildner, Penetrationsförderer, Glanzmittel, Aromastoffe, Detergenzien, Schmierstoffe oder Geschmacksmittel sein. Sie dienen als Verarbeitungshilfsmittel und sollen ein sicheres und reproduzierbares Herstellungsverfahren sowie gute Langzeitlagerstabilität gewährleisten oder sie erreichen in der Arzneiform zusätzliche vorteilhafte Eigenschaften. Sie werden den Polymerzubereitungen vor der Verarbeitung zugesetzt und können die Permeabilität der Überzüge beeinflussen, was ggf. als zusätzlicher Steuerparameter genutzt werden kann.

• Trennmittel:

Trennmittel besitzen in der Regel lipophile Eigenschaften und werden in der Regel den Sprühsuspensionen zugesetzt. Sie verhindern eine Agglomeration der Kerne während der Befilmung. Bevorzugt werden Talkum, Mg- oder Ca - Stearat, gemahlene Kieselsäure, Kaolin oder nicht ionische Emulgatoren mit einem HLB - Wert zwischen 3 und 8 eingesetzt. Übliche Einsatzmengen für Trennmittel in den erfindungsgemäßen Überzugs- und Bindemitteln liegen zwischen 0,5 bis 100 Gew.-% bezogen auf das Copolymer.

• Pigmente:

Mit dem Überzugsmittel unverträgliche Pigmente sind insbesondere solche Pigmente, die wenn sie der (Meth)acrylat-Copolymer-Dispersion direkt zugesetzt werden, z. B. durch Einrühren, in üblichen Anwendungsmengen von z. B. 20 bis 400 Gew.-% bezogen auf das Trockengewicht des (Meth)acrylat-Copolymeren zur Destabilisierung der Dispersion, Koagulation, zu Entmischungserscheinungen oder ähnlich unerwünschten Effekten führen. Weiterhin sind die zu verwendenden Pigmente natürlich nicht toxisch und für pharmazeutische Zwecke geeignet. Siehe dazu z. B. auch: Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980.

Mit dem Überzugsmittel unverträgliche Pigmente können z. B. Aluminiumoxidpigmente sein. Unverträgliche Pigmente sind z. B., Gelborange, Cochenillerotlack, Farbpigmente auf Basis von Aluminiumoxid bzw Azofarbstoffen, Sulfonsäurefarbstoffe, Gelborange S (E110, C.I. 15985, FD&C Yellow 6), Indigocarmin (E132, C.I. 73015, FD&C Blue 2), Tartrazin (E 102, C.I. 19140, FD&C Yellow 5), Ponceau 4R (E 125, C.I. 16255, FD&C Cochineal Red A), Chinolingleb (E 104, C.I. 47005, FD&C Yellow 10), Erythrosin (E127, C.I. 45430, FD&C Red 3), Azorubin (E 122, C.I. 14720, FD&C Carmoisine), Amaranth (E 123, C.I. 16185, FD&C Red 2), Brilliantsäuregrün (E 142, C.I. 44090, FD&C Green S).

Die angegebenen E-Nummern der Pigmente beziehen sich auf eine EU-Nummerierung. Siehe dazu auch "Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980. Die FD&C-Nummern beziehen sich auf die Zulassung in Food, Drugs und Cosmetics durch U.S. Food and Drug Administration (FDA) beschrieben in: U.S. Food and Drug Administration, Center for Food Safety and Applied Nutrition, Office of Cosmetics and Colors: Code of Federal Regulations - Title 21 Color Additive Regulations Part 82, Listing of Certified Provisionally Listed Colors and Specifications (CFR 21 Part 82).

• Weichmacher

Weitere Zuschlagstoffe können auch Weichmacher sein. Übliche Mengen liegen zwischen 0 und 50, bevorzugt 2 bis 20, insbesondere 5 bis 10 Gew.-%.

Weichmacher können je nach Typ (lipophil oder hydrophil) und zugesetzter Menge die Funktionalität der Polymerschicht beeinflussen. Weichmacher erreichen durch physikalische Wechselwirkung mit dem Polymeren eine Absenkung der Glasübergangstemperatur und fördern in Abhängigkeit von der zugesetzten Menge die Verfilmung. Geeignete Stoffe haben in der Regel ein Molekulargewicht zwischen 100 und 20.000 und enthalten eine oder mehrere hydrophile Gruppen im Molekül, z. B. Hydroxyl-, Ester- oder Aminogruppen.

Beispiele geeigneter Weichmacher sind Citronensäurealkylester, Glycerinester, Phthalsäurealkylester, Sebacinsäurealkylester, Succroseester, Sorbitanester, Diethylsebacat, Dibutylsebacat und Polyethylenglykole 200 bis 12.000. Bevorzugte Weichmacher sind Triethylcitrat (TEC) und Acetyltriethylcitrat (ATEC). Weiterhin zu nennen sind in der Regel bei Raumtemperatur flüssige Ester wie Citrate, Phthalate, Sebacate oder Rizinusöl. Bevorzugt werden Zitronensäure- und Sebacinsäureester verwendet.

Die Zugabe der Weichmacher zur Formulierung kann in bekannter Weise, direkt, in wäßriger Lösung oder nach thermische Vorbehandlung der Mischung vorgenommen werden. Auch können Mischungen von Weichmachern eingesetzt werden

**Herstellung multipartikulärer Arzneiformen**

Die wirkstoffhaltigen, überzogenen Pellets können mittels pharmazeutisch üblicher Hilfsstoffe und in an sich bekannter Weise zu multipartikulären Arzneiformen, insbesondere zu pellethaltigen Tabletten, Minitabletten, Kapseln, Sachets oder Trockensäften verarbeitet werden, die so formuliert sind, daß die enthaltenen Pellets im pH-Bereich des Magens freigesetzt werden. Die Darstellung als multipartikuläre Arzneiform stellt eine hohe Dosiersicherheit bietet den Vorteil einer guten Verteilung der Pellets im Darmlumen. Die erfindungsgemäße multipartikuläre Arzneiform kann zudem auch verschiedene Pellettypen mit unterschiedlichen Wirkstoffen und/oder unterschiedlichem Pellet-Aufbau enthalten.

<u>Verpreßte Tabletten</u>

Die Herstellung von multipartikulären Arzneiformen durch Verpressen eines pharmazeutisch üblichen Bindemittels mit wirkstoffhaltigen Partikeln ist z. B. Beckert et al. (1996), "Compression of enteric-coated pellets to disintegrating tablets", International Journal of Pharmaceutics 143, S. 13 - 23, und in WO 96/01624 beschrieben.

Filmüberzüge auf wirkstoffhaltige Pellets werden üblicherweise in Wirbelschichtgeräten aufgebracht. Rezepturbeispiele sind in dieser Anmeldung erwähnt. Filmbildner werden üblicherweise mit Weichmachern und Trennmitteln nach einem geeigneten Verfahren gemischt. Hierbei können die Filmbildner als Lösung oder Suspension vorliegen. Die Hilfsstoffe für die Filmbildung können ebenfalls gelöst oder suspendiert sein. Organische oder wässrige Löse- oder Dispergiermittel können verwendet werden. Zur Stabilisierung der Dispersion können zusätzlich Stabilisatoren verwendet werden (Beispiel: Tween 80 oder andere geeignete Emulgatoren bzw. Stabilisatoren).

Beispiele für Trennmittel sind Glycerolmonostearat oder andere geeignete Fettsäurederivate, Kieselsäurederivate oder Talkum. Beispiele für Weichmacher sind Propylenglykol, Phthalate, Polyethylenglykole, Sebacate oder Citrate, sowie andere in der Literatur erwähnte Substanzen.

Zwischen wirkstoffhaltiger und darmlöslicher Copolymer-Schicht kann eine trennende Schicht aufgebracht sein, die der Trennung von Wirkstoff und Überzugsmaterial zum Zwecke der Verhinderung von Interaktionen dient. Diese Schicht kann aus inerten Filmbildnern (z.B. HPMC, HPC oder (Meth)acrylsäure-Copolymeren) oder z.B. Talkum oder einer anderen geeigneten pharmazeutischen Substanzen bestehen. Ebenso können Kombinationen aus Filmbildnern und Talkum oder ähnlichen Stoffen verwendet werden. Es ist auch möglich, eine Trennschicht aus teilweise bzw. vollneutralisierten (Meth)acrylatcopolymer-Dispersionen aufzubringen.

Die Trennschicht kann auch aus dem gleichen oder einem anderen mucoadhaesiven Polymer wie in der unterliegenden Matrixschicht bestehen. Auf diese Weise kann eventuellen Interaktionen oder Unverträglichkeiten des Wirkstoffs oder des mucoadhaesiven Polymers mit der filmbildenden (Meth)acrylatcopolymer-Schicht begegnet werden.

Mischungen zur Herstellung von Tabletten aus überzogenen Partikeln werden durch Vermischen der Pellets mit geeigneten Bindemitteln für die Tablettierung, nötigenfalls der Zugabe von zerfallsfördernden Substanzen und nötigenfalls der Zugabe von Schmiermitteln zubereitet. Das Mischen kann in geeigneten Maschinen stattfinden. Ungeeignet sind Mischer, die zu Schäden an den überzogenen Partikeln führen, z. B. Pflugscharmischer. Zur Erzielung geeigneter kurzer Zerfallszeiten kann eine spezielle Reihenfolge bei der Zugabe der Hilfsstoffe zu den überzogenen Partikel erforderlich sein. Durch Vormischung mit der überzogenen Partikel mit dem Schmier- oder Formentrennmittel Magnesiumstearat kann dessen Oberfläche hydrophobisiert und somit Verkleben vermieden werden.

Zum Tablettieren geeignete Mischungen enthalten üblicherweise 3 bis 15 Gew.-% eines Zerfallshilfsmittels, z. B. Kollidon CL und z. B. 0,1 bis 1 Gew.-% eines Schmier- und Formentrennmittels wie Magnesiumstearat. Der Bindemittelanteil bestimmt sich nach dem geforderten Anteil an überzogenen Partikeln.

Typische Bindemittel sind z. B. Cellactose®, mikrokristalline Cellulose, Calciumphosphate, Ludipress®, Lactose oder andere geeignete Zucker, Calciumsulfate oder Stärkederivate. Bevorzugt werden Substanzen mit geringer Schüttdichte.

Typische Zerfallshilfsmittel (Sprengmittel) sind quervernetzte Stärke- oder Cellulosederivate, sowie quervernetztes Polyvinylpyrrolidon. Ebenso sind Cellulosederivate geeignet. Durch Auswahl eines geeigneten Bindemittels kann die Verwendung von Zerfallshilfsmittel entfallen.

Typische Schmier- und Formentrennmittel sind Magnesiumstearate oder andere geeignete Salze von Fettsäuren oder in der Literatur zu diesem Zweck aufgeführte Substanzen (z.B. Laurinsäure, Calciumstearat, Talkum usw.). Bei Verwendung geeigneter Maschinen (z.B. Tablettenpresse mit externer Schmierung) oder geeigneter Formulierungen kann die Verwendung eines Schmier- und Formentrennmittels in der Mischung entfallen.

Der Mischung kann gegebenenfalls ein Hilfsmittel zur Fließverbesserung beigefügt sein (z. B. hochdisperse Kieselsäurederivate, Talkum usw.).

Das Tablettieren kann auf üblichen Tablettenpressen, Exzenter- oder Rundlauftablettenpressen erfolgen, bei Preßkräften im Bereich von 5 bis 40 kN, bevorzugt 10 - 20 kN. Die Tablettenpressen können mit Systemen zur externen Schmierung ausgestattet sein. Gegebenenfalls kommen spezielle Systeme zur Matrizenbefüllung zum Einsatz, die die Matrizenbefüllung mittels Rührflügeln vermeiden.

Weitere multipartuläre Arzneiformen

Alternativ zu verpressten Tabletten bzw. Minitabletten, können die wirkstoffhaltigen, überzogenenen Pellets auch zu beliebige anderen, oral verabreichbareb multipartikuläre Arzneiformen verarbeitet werden. Die überzogenen Pellets können z. B. in Kapseln, z. B. Gelatinekapsel, verfüllt werden oder zu Sachets oder Trockensäfte formuliert werden.

**Vorteilhafte Wirkungen der Erfindung**

Die erfindungsgemäße Arzneiform eignet sich zur gezielten und effektiven Freisetzung von Protein- oder Peptidwirkstoffen. Die Arzneiform weist eine hohe Dosiersicherheit und verteilt sich gut im Darmlumen. Der enthaltene Protein- oder Peptidwirkstoff wird dabei weitgehend gegenüber physikalischer oder proteolytischer Inaktivierung geschützt und kann am definierten Wirkort so freigesetzt werden, daß ein hoher Anteil des Wirkstoffs von Körper aufgenommen werden kann. Die Arzneiform kommt daher mit weniger Wirkstoff aus, da nur wenig Wirkstoff verloren geht. Die Gefahr von Nebenwirkungen wird durch die gezielte Abgabe insgesamt verringert. Der Wirkort kann je nach therapeutischem Ziel variabel eingestellt werden. Der Zeitpunkt der Wirkstoffaufnahme kann somit besser gesteuert werden. Da es sich um eine orale Arzneiform handelt hat diese eine insgesamt bessere Akzeptanz der Patienten ("patient compliance") im Vergleich zu anderen Applikationsformen. Eine Vielzahl von Peptid- oder Proteinwirkstoffe wird dadurch erstmals der peroralen Anwendung zugänglich und haben somit weniger Applikationsrisiken wie insbesondere bei parenteralen Applikationen. Auch die Kosten der Applikation können gering gehalten werden, da kein Fachpersonal für die Applikation notwendig ist.

Eine beschleunigte Freigabe bei gleichzeitiger Steigerung der Bioverfügbarkeit kann erreicht werden aus Matrixsystemen, in denen der Anteil des Polymeren mit mucoadhaesiver Wirkung in Gew.-% 3-fach, bevorzugt 1000-fach höher ist als der Wirkstoffanteil.

**Lipophile Matrix**

Ein besonderer Aspekt der Erfindung ergibt sich wenn der Wirkstoff in eine lipophile Matrix eingebettet ist, die einen Schmelzpunkt oberhalb von 37 °C, bevorzugt oberhalb von 45 °C, besonders bevorzugt bevorzugt oberhalb von 55 °C aufweist und wirkstoffhaltige lipophile Matrix in die Matrix aus dem Polymeren mit mucoadhaesiver Wirkung eingebettet ist. Die Formulierung in der lipophilen Matrix zielt darauf ab, die Löslichkeit bzw. die Bioverfügbarkeit des Wirkstoffs, bevorzugt von wenig oder schwerlöslichen Wirkstoffen (im Sinne der DAB 10, 2003) zu verbessern.

Unter einer lipophilen Matrix im Sinne der Erfindung versteht man eine Substanz oder eine Mischung von Substanzen, in der oder denen der Wirkstoff gelöst, suspendiert oder emulgiert werden kann. Die Substanz oder die Substanzen der lipophilen Matrix sind verschieden von den üblichen pharmazeutischen Hilfsstoffen und dem Polymer mit mucoadhaesiver Wirkung. Die Substanz oder die Substanzen der lipophilen Matrix haben bevorzugt einen hydrophoben oder auch amphiphilen Charakter. Man könnte die lipophile Matrix auch als amphiphile Matrix oder als lipoide Matrix bezeichnen.

Die lipophile Matrix kann aus einer einzelnen Substanz, z. B. einem Lipid, oder einer Mischung von Substanzen, z. B einer Mischung von Lipiden, bestehen. Im Falle von Mischungen errechnen sich die im folgenden beschriebenen Eigenschaften für Wasserlöslichkeiten nach DAB 10, Partitionskoeffizienten und/oder HLB-Werte jeweils aus dem arithmetrischen Mittel aus den Gewichtsteilen und den Werten der Substanzen der Mischung. Die eingesetzten Substanzen dürfen nicht toxisch sein.

Der Wirkstoff und die Substanz oder die Substanzen, die die lipophile Matrix bilden, unterscheiden sich bevorzugt in ihrer Löslichkeit in Wasser nach DAB 10 und nicht mehr als +/- 50 %, bevorzugt nicht mehr als +/- 25 % unterscheiden und/oder in ihrem Verteilungskoeffizienten nach Anhang V zu RL 67/548/EWG, A.8 nicht mehr als +/- 60 %, bevorzugt nicht mehr als +/- 30 % unterscheiden und/oder in ihrem HLB-Wert, sofern ein den Substanzen ein HLB Wert zugeordnet werden kann, gemessen nach Marszall nicht mehr als +/- 80 %, bevorzugt nicht mehr als +/- 40 % unterscheiden. Je höher die Übereinstimmung des Wirkstoff mit der lipophilen Matrix in zumindest einer, bevorzugt zweier oder allen drei der genannten Eigenschaften, desto mehr ist die Löslichkeit und die Bioverfügbarkeit des Wirkstoffs in der Arzneiform begünstigt.

### Löslichkeit in Wasser

Die Löslichkeit in Wasser für den Wirkstoff und die Substanz oder die Substanzen, die die lipophile Matrix bilden, kann nach DAB 10 (Deutsches Arzneibuch, 10. Ausgabe mit 3. Nachtrag 1994, Deutscher Apothekerverlag, Stuttgart und Govi Verlag, Frankfurt a. M., 2. Nachtrag (1993), IV Allgemeine Vorschriften, S. 5 - 6, "Löslichkeit und Lösungsmittel"; s. a. Ph. Eur. 4.07, 2004), definiert werden. Die Definition der Löslichkeit erfolgt über die Anzahl der Volumenteile Lösungsmittel für 1 Gewichtsteil Substanz, bzw. Arzneistoff. Die Definition "wenig löslich" umfasst Substanzen, die über 30 bis 100 Volumenteile Lösungsmittel für 1 Gewichtsteil Substanz, bzw. Arzneistoff erfordern, die Definition "schwer löslich" umfasst Substanzen, die über 100 bis 1000 Volumenteile Lösungsmittel für 1 Gewichtsteil Substanz, bzw. Arzneistoff erfordern.

### Verteilungskoeffizienten

Die Verteilungskoeffizienten für den Wirkstoff und die Substanz oder die Substanzen, die die lipophile Matrix bilden, können nach Anhang V zu RL 67/548/EWG, A.8 "Verteilungskoeffizient" bestimmt werden.

### HLB-Wert

Der HLB-Wert ist ein 1950 von Griffin eingeführtes Maß der Hydrophilie bzw. Lipophilie von nichtionischen Tensiden. Er läßt sich experimentell durch die Phenol-Titrationsmethode nach Marszall bestimmen; vgl. "Parfümerie, Kosmetik". Band 60, 1979, S. 444 - 448; weitere Literaturhinweise in Römpp, Chemie-Lexikon, 8.Aufl. 1983, S.1750. Siehe weiterhin z. B. US 4 795 643 (Seth)). Ein HLB-Wert (Hydrophile/Lipophile Balance) läßt sich nur bei nicht ionischen Substanzen exakt bestimmen. Bei anionischen Substanzen kann dieser Wert rechnerisch ermittelt werden, liegt jedoch praktisch immer über oder weit über 14.

HLB-Werte für den Wirkstoff und die Substanz oder die Substanzen, die die lipophile Matrix bilden, können in den meisten Fällen nach Marszall bestimmt werden, aus Tabellen pharmazeutischer oder chemischer Nachschlagewerke oder Lehrbücher entnommen werden oder nach bei ionischen Substanzen rechnerisch ermittelt werden.

### Wirkstoffe in der lipophilen Matrix

Bevorzugt enthält die Arzneiform in der lipophilen Matrix einen Wirkstoff, der eine Löslichkeit in Wasser nach DAB 10 von mindestens 30, insbesondere über 30 bis 100 oder über 100 bis 1000 Volumenteilen Wasser für einen Gewichtsteil Wirkstoff aufweist. Der bevorzugte Wirkstoff ist demnach wenig oder sogar schwer löslich nach der Definition der DAB 10.

Der in der lipophilen Matrix fomulierte Wirkstoff kann aus z. B. aus der Gruppe der Peptidantibiotika, Immunsupressiva, LHRH-Antagonisten, Immunomodulatoren ausgewählt werden.

Der in der lipophilen Matrix fomulierte Wirkstoff kann z. B. Abarelix, Angiotensin II, Anidulafungin, Antide, Argipressin, Azalin und Azalin B, Bombesin-Antagonist, Bradykinin, Buserelin, Calcitonin, Cetrorelix, Ciclosporin, Ciclosporin A, Desmopressin, Detirelix, Erytropoietin, Enkephaline (Leu-, Met-) Ganirelix, Gonadorelin, Goserelin, Growinnormone-Secretagogue, Insulin, Interferon (alpha, beta, gamma), Interleukine (IL1, IL2). Micafungin, Nafarelin, Leuprolide, Leuprorelin, Octreotid, Orntide, Oxytocin, Parathyroid Hormon, Ramorelix, Sekretin, Somatotropin, Terlipressin, Tetracosactid, Teverelix, Triptorelin, Thyroliberin, Thyrotropin Tumornekrosefaktor (TNF alpha, beta),oder Vasopressin sein.

**Lipophile Matrix/Polymere mit mucoadhaesiver Wirkung**

In einer bevorzugt Ausführungsform werden mögliche Interaktionen der lipophile Matrix mit dem Polymer mit mucoadhaesiver Wirkung berücksichtigt. Um nicht kontrollierbare Wechselwirkungen zu vermeiden soll die Substanz oder die Substanzen, die die lipophile Matrix bilden, und das Polymere mit mucoadhaesiver Wirkung bevorzugt entweder die gleichen ionische Eigenschaften aufweisen, d.h. beide sollen übereinstimmend entweder zumindest überwiegend kationischen oder übereinstimmend anionischen Charakter haben. Im Falle, daß die Substanzen mit entgegengesetzten ionischer Eigenschaften ausgewählt werden, soll das Polymere mit mucoadhaesiver Wirkung bevorzugt zu mindestens 50, besonders bevorzugt zu 100 % in neutralisierter Form vorliegen. Die Neutralisation kann durch Zusatz von Säure oder Base in bekannter Weise erfolgen.

**Substanz oder Substanzen für den Aufbau der lipophilen Matrix**

Bevorzugt besteht die lipophile Matrix zu 80 bis 100, bevorzugt zu 90 bis 100, besonders bevorzugt zu 100 Gew.-% aus einer Substanz oder einer Mischung von Substanzen mit einem (gemittelten) HLB-Wert von 0 bis 15, bevorzugt 2 bis 10 besteht. Die lipophile Matrix kann 0 bis 20, bevorzugt 0 bis 10 Gew.-% an pharmazeutisch üblichen Hilfsstoffen, insbesondere Stabilisatoren, Verdickungsmittel oder Adsorbentien, enthalten. Besonders bevorzugt sind keine pharmazeutisch üblichen Hilfsstoffe enthalten.

Die Substanz oder die Substanzen, die die lipophile Matrix bilden, können z. B. zu der Gruppe der Öle. Fette, Mono-, Di- oder Triglyceride. Fettsäuren, Fettalkohole, insbesondere $C_6$- bis $C_{20}$-Fettsäure und/oder einen $C_6$- bis $C_{20}$-Alkohol einschließlich deren Salze, Ether-, Ester- oder Amid-Derivaten, Phospholipide, Lecithine, Emulgatoren, Lipoide, lipidlösliches Vitamine oder Tenside gehören.

Die lipophile Matrix kann z. B. eine der folgenden Lipidpräparationen enthalten: (Imwitor 308) Glycerylmonocaprylate mit einem Monoesteranteil >80 %, (Imwitor 312) Glycerylmonolaurate mit enem Monoesteranteil > 90 %, (Imwitor 491) Glycerolmonosterate ($C_{16}$ + $C_{18}$) mit einem Monoesteranteil >90 %, (Imwitor 900 P) Glycerolmonosterat mit einem Monoesteranteil von 40 - 55% und einem $C_{18}$-Gehalt von 40 - 60%, (Imwitor 900 K) Glycerolmonosterat, mit einem Monoesteranteil von 40 - 55 % und einem $C_{18}$-Gehalt von 60 -80 %, (Imwitor 742) Mittelkettige $C_8$ und $C_{10}$ Glyceride mit einem Monoesteranteil von 45 - 55 %, (Imwitor 928) Partielle Glyceride gesättigter pflanzlicher $C_{10}$-$C_{18}$Fettsäuren mit einem Hauptanteil an $C_{12}$, und mit einem Monoesteranteil von 34-36%, $C_8$ and $C_{10}$-Glyceride, NaCaprylat oder NaCapriat.

Die lipophile Matrix kann z. B. eine der folgenden Lipidpräparationen enthalten: Fette wie Mono-, Di-, Triglyceride von gesättigten und ungesättigten Fettsäuren und deren Mischungen. Insbesondere Glycerin-Stearinsäureester, Glycerin-Palmitinsäureester, Glycerin-Myristinsäureester, Glycerin-Palmitinsäure-Stearinsäureester, Glycerin-Laurinsäureester Glycerin-Caprylsäureester, Glycerin-Ölsäureester, Beispiele für diese Ester sind Imwitor® - 308, - 312, - 491, 742, - 900, - 928, - 988, sowie Gelucire ® 44/14, - 50/13, Geleol, Compritol E ATO, Dynasan 114, Softisan, Witepsol, Dynacet 212, Kokosfett.

Öle wie beispielsweise Rizinusöl, Sesamöl, Sonnenblumenöl, Baumwollsamenöl, Maisöl, Mandelöl, Erdnussöl, Olivenöl, Kokosnussöl, Karottenöl, Weizenkeimöl, Walnussöl.

Neutralöle wie Isopropylmyristat, -palmitat, -stearat, mittelkettige Triglyceride (Miglyol®).

Kurzkettige aliphatische und aromatische Carbonsäureester wie beispielsweise Dibutylphthalat, Diethylsebacat, Dibutylsebacat, Tributylcitrat, Acetyltributylcitrat, Glycerintriacetat.

Wachse wie beispielsweise Kanaubawachs, Bienenwachs, Wollwachs Glycerinbehensäureester.

Fettsäureamide wie beispielsweise Stearinsäureamid, Palmitinsäureamid, Laurinsäureamid.

Aliphatische langkettige Carbonsäuren wie beispielsweise Stearinsäure, Palmitinsäure, Laurinsäure, Myristinsäure, Ölsäure, Caprylsäure, Linolsäure, Linolensäure. Sowie beispielsweise deren Na-, Al- und Mg-Salze.

Fettalkohole wie beispielsweise Stearylalkohol, Laurytalkohol, Cetylalkohol, Myristinalkohol, Glycerinformal.

W/O Emulgatoren wie beispielsweise Cholesterol, Glycerinmonostearat, Ethylenglycolmonostearat, Sorbitanmonooleat (Span® 80), -palmitat (Span@ 40), -laurat (Span@ 20), -stearat (Span@ 60), Sorbitantrioleat (Span® 85)" Sorbitantristearat (Span@ 65), Sorbitansesquioleate (Arlacel® 83), Ca-, Al, Mg-Stearat, Polyoxethylensorbitantristearat (Tween® 65), Polyoxethylensorbitantrioleat (Tween® 85).

Nichtionische O/W Emulgatoren wie beispielsweise Macrogolstearat 400 (Chremophor® A), Macrogollaurylether, Polyethylenglykol-20-Sorbitanmonolaurat, -stearat, -palmitat, -oleat, Macrogol-1500-glycerintriricinoleat, Macrogolglycerinhydroxy-stearat (Cremophor® RH), Macrogol-1000-glycerinmono-laurat, -stearat, -oleat, Saccharosemonostearat. Polysorbat 60 (Tween® 60), Polyoxyethylenmonostearat (Myrj 49), Polysorbat 80 (Tween® 80), Polysorbat 40 (Tween® 40), Polysorbat 20 (Tween® 20), Poloxamer 407 (Lutrol® F 127), Poloxamer 188 (Lutrol® F 68), Polyoxyethlylenrizinoleat (Cremophor® EL), Polyoxyethylen-5-stearylstearat.

Ionische O/W Emulgatoren wie beispielsweise Cetylstearylsulfat (Lanette® E), Na-Laurylsulfat (Texapon® Z), Na-Glycocholat, Hederagenin.

Amphiphile Emulgatoren wie beispielsweise Ei-Phosphatidylcholin (Eilecithin), Soja-Phosphatidylcholin (Sojalecithin), Betain, Sulfobetaine, Ceramide (Sphingomyelin).

Vitamine wie beispielsweise Retinol (Vitamin A), Cholecalciferol (Vitamin D), alpha-Tocopherol und alpha-Tocopherolacetat (Vitamin E), Phyllochinon (Vitamin K).

Weitere Hilfsstoffe sind Galaktolipide wie beispielsweise Monogalaktosyldiacylglycerin, Digalaktosyl-diacylglycerin, Trigalaktosyl-diacylglycerin, sowie aromatische Öle wie beispielsweise Anisöl, Citronellöl, Eukalyptusöl, Fenchelöl, Kamillenöl, Kardamomenöl, Kiefemadelöl, Kümmelöl, Latschenöl, Lavendelöl, Minzöl, Muskatöl, Nelkenöl, Pfefferminzöl, Rosmarinöl, Salbeiöl und Terpene wie beispielsweise Menthol, Linalool, 1,4-Cineol, Pyrethrin, Borneol, Eudesmol, Phytol, Manool, Azadirachtin, Nimbin.

Bevorzugt ist der Wirkstoff zu mindestens 10 %, besonders bevorzugt zu mindestens 20 %, insbesondere zu mindestens 50 % in der lipophilen Matrix löslich.

Der Gehalt der wirkstoffhaltigen Lipidmatrix an der inneren Matrix-Schicht a) kann 1 bis 50, bevorzugt 10 bis 20 Gew.-% betragen.

Bevorzugt enthält die iipophiie Matrix zu mindestens 50 Gew.-% Glycerinmonocaprylat, bis zu 10 Gew.-% Na-Cholat, bis zu 10 Gew.-% Tocopherol-Succinat, 1 bis 5 Gew.-% eines Efflux-Pumpen-Inhibitors im Falle, daß der Wirkstoff ein Substrat der PgP-Effluxpumpe ist, z. B. Solutol HS 15, ein Triglycerid, insbesondere Tristearat, wobei sich die Komponenten zu 100 % addieren. Diese lipophile Matrix kann direkt ins mucoadhaesive Polymer eingearbeitet werden oder in Wasser emulgiert in das mucoadhaesive Polymer eingearbeitet werden. Im letzteren Fall kann die Wasserphase eine schwache Säure, wie z. B. Citronensäure, beinhalten.

Bei wenig oder noch schlechter löslichen (nach DAB 10) Wirkstoffen mit einem Molekulargewicht > 3.000, ist ein Proteaseinhibitor, wie z.B. Sojabohnentrysininhibitor, in der wäßrigen Phase enthalten.

## Verfahren

Die Erfindung betrifft auch ein Verfahren zur Herstellung einer multipartikulären Arzneiform mit den Schritten

a) Erzeugen der wirkstoffhaltigen lipophilen Matrix durch Suspendieren und/oder Lösen des Wirkstoffs mit der oder den Substanzen, die die lipohile Matrix bilden und gegebenenfalls weiteren pharmazeutisch üblichen Hilfsstoffen durch intensives Mischen oder Schmelzen der Inhaltsstoffe,

b) Erzeugen von Präpellets (Pelletkerne) mittels Sprühauftrag des mucoadhaesiven Polymers in Mischung mit der wirkstoffhaltigen lipophilen Matrix auf einen Kern oder durch Rotagglomeration, Ausfällen oder Sprühverfahren ohne einen Kern,

c) Erzeugen von Pellets durch Sprühauftrag eines Überzuges des anionischen Polymers oder Copolymers, das optional Beimengungen pharmazeutisch üblicher Hilfsstoffe, insbesondere Weichmachern und Trennmittel, enthalten kann, aus einer Dispersion oder organischer Lösung auf die Präpellets aus Schritt b),

d) Herstellung einer multipartikulären Arzneiform, durch Verfüllen oder Einarbeiten der Pellets aus Schritt c) in an sich bekannter Weise, gegebenenfalls unter Verwendung pharmazeutisch üblicher Hilfsstoffe, insbesondere durch Verarbeitung zu pellethaltigen Tabletten, Minitabletten, Kapseln, Sachets oder Trockensäften.

## Bevorzugtes Verfahren

Bevorzugt werden die Verfahrensschritte a) und b) wie folgt ausgeführt:

a) Erzeugen der inneren Matrix-Schicht durch Herstellen einer Emulsion, Dispersion oder Lösung des Wirkstoffs mit der oder den Substanzen für die lipophilen Matrix, und gegebenenfalls weiteren pharmazeutisch üblichen Hilfsstoffen durch intensives Mischen der Inhaltsstoffe in Wasser und Erzeugung einer Öl in Wasser-Präparation mit einer mittleren Teilchengröße von nicht mehr als 60, bevorzugt nicht mehr als 20 μm,

b) Erzeugen von Präpellets mittels Sprühauftrag der Öl in Wasser-Präparation aus Schritt a) auf das mucoadhaesive Polymer, das gegebenenfalls Beimengungen weiterer pharmazeutisch übliche Hilfsstoffe enthalten kann, wobei die Inhaltstoffe in Form eines mikronisierten Pulvers, z. B. mit einer mittleren Korngröße von 10 bis 100 μm, vorliegen, durch Rotagglomeration, Extrusion oder Granulation.

## BEISPIELE

Herstellung von Pellets, enthaltend mucoadhaesiv formulierte Peptid- und Protein-Wirkstoffe

### Vergleichsbeispiel 1

Erste Beschichtung (Präpellets):

20 g Na-Carboxymethylcellulose (Blanose 7LF, Hercules-Aualon) = 10% bezogen auf die Pellets (Wasseraufnahme Blanose 7LF: ca. 50 % in 15 min bei pH 7,2 in Phosphatpuffer, Mucoadhäsivität bei pH 7,2 in gemessen nach Hassan u. Gallo: $\eta_b$=ca. 250 mPa.s) werden zusammen mit 1,25 g Aerosil 200 (mikrokristalline Cellulose) = 6,25% bezogen auf Blanose in 378,8 g demineralisiertem Wasser unter Rühren mit einem Propellerrührer gelöst. 0,72 g Polysorbat 80 (33%ig) =40% bezogen auf Glycerinmonostearat (GMS) werden unter Rühren in 10g Wasser gelöst. Nach Zugabe von 0,6 g GMS = 3% bezogen auf Blanose und demineralisiertem Wasser ad 20 g wird die Dispersion auf 80°C erhitzt. Die Dispersion wird nach Abkühlen auf 30°C mit dem Ultraturrax-Mischer 10 min homogenisiert und anschließend zur Blanoselösung unter Rühren gegeben. Anschließend werden 139,4 mg Desmopressin-acetat ($M_w$ = 1067) = 0,062% in der Formulierung in 30 g demin. Wasser gelöst und zur Blanose-Lösung gegeben.

200 g non pareil pellets 850 -1000 μm werden im Mini-Wirbelschichtgerät (MiniGlatt, Fa. Glatt, Binzen) vorgelegt und mit der Desmopressin-Blanose-Lösung beschichtet.

Sprühparameter:
Sprühdüse 0,5 mm
Sprührate 1-1,26 g/min
Sprühdruck 0,8 bar
Zuluftdruck 1 bar
Zulufttemperatur 45°C
Produkttemperatur 41,5-43°C
Nachtrocknung im Mini Glatt 10 min bei 40°C
Sprühzeit: ca. 2 bis 6 h
Trocknung über Nacht bei RT
Schichtdicke (REM): 12-18 μm

Tabelle 1: Freigabe (nach USP XXV) von Desmopressin in Phosphatpuffer pH 7,2; 100 min$^{-1}$; Paddle; 1h; 100 rpm; 37°C; n=4; (erste Schicht). Das freigewordene Desmopression wird mit dem Spektrometer bei 220 nm detektiert.

| Zeit [min] | 0 | 5 | 10 | 15 | 20 | 25 | 30 | 45 | 60 |
|---|---|---|---|---|---|---|---|---|---|
| Muster 1 [%] | 0,0 | 54,9 | 74,7 | 79,4 | 83,8 | 87,8 | 94,4 | 100,3 | 103,8 |
| Muster 2 [%] | 0,0 | 46,8 | 58,4 | 76,3 | 79,5 | 82,0 | 88,0 | 97,3 | 94,3 |
| Muster 3 [%] | 0,0 | 53,6 | 65,0 | 77,9 | 81,7 | 89,6 | 91,6 | 92,4 | 97,4 |
| Muster 4 [%] | 0,0 | 55,6 | 72,5 | 80,2 | 86,9 | 90,4 | 96,3 | 94,7 | 100,3 |
| Mittelwert [%] | 0,0 | 52,7 | 67,7 | 78,4 | 83,0 | 87,4 | 92,6 | 96,2 | 99,0 |

(fortgesetzt)

| Zeit [min] | 0 | 5 | 10 | 15 | 20 | 25 | 30 | 45 | 60 |
|---|---|---|---|---|---|---|---|---|---|
| StdAbw. [%] | 0,0 | 4,0 | 7,5 | 1,7 | 3,2 | 3,8 | 3,6 | 3,4 | 4,0 |

**Versgleichsbeispiel 2**

Zweite Beschichtung (Pellets):

66,7 g Eudragit® FS30D (30-%ige Dispersion, enhaltend ein Copolymer aus 25 Gew.-%, Methylmethacrylat, 65 Gew.-% Methylacrylat und 10 Gew.-% Methacrylsäure, Röhm GmbH & Co.KG, Darmstadt) werden mit 1 g Triethylcitrat (TEC) = 5% bezogen auf LTS (Lacktrockensubstanz) in einem 150 mL Becherglas gemischt. 2,2 g Polysorbat 80 (33%ig) = 40% bezogen auf GMS werden unter Rühren in 46 g demineralisiertes Wasser gelöst. Nach Zugabe von 1,8 g GMS = 9% bezogen auf LTS und demineralisiertem Wasser wird die Dispersion auf 80°C erhitzt. Die Dispersion wird nach Abkühlen auf 30°C mit dem Ultraturrax 10 min homogenisiert und anschließend unter Rühren zur Eudragit® FS30D Dispersion gegeben. Nach 30 min Rühren werden damit 100 g der Desmopressin - Blanose beschichteten Pellets aus Beispiel 1 im MiniGlatt vorgelegt und mit der Eudragit® FS30D Dispersion beschichtet.

Sprühparameter:

Sprühdüse 0,5 mm
Sprührate 0,6 - 0,9 g/min
Sprühdruck 0,7 bar
Zuluftdruck 0,7 bar
Zulufttemperatur 30°C
Produkttemperatur 29 -30°C
Nachtrocknung im Mini Glatt 10 min bei 40°C
Trocknung über Nacht bei RT
Sprühzeit: ca. 1 bis 2,5 h
Schichtdicke (REM): 40-45 $\mu$m,

Tabelle 2: Desmopressin Freigabe von EUDRAGIT® FS 30D beschichteten Pellets, 2 h in 0.1 M HCl, 1 h in Phosphatpuffer pH 7.2; 100 rpm; Paddle; 37°C; n=4 (zweiter Überzug)

| Zeit [min] | 0 | 60 | 120 | 125 | 130 | 140 | 145 | 160 | 170 | 180 |
|---|---|---|---|---|---|---|---|---|---|---|
| Muster 1 | 0,0 | 0,0 | 0,0 | 0,0 | 0,0 | 62,7 | 75,1 | 91,3 | 93,4 | 94,1 |
| Muster 2 | 0,0 | 0,0 | 0,0 | 0,0 | 0,0 | 57,1 | 71,0 | 91,4 | 93,2 | 97,1 |
| Muster 3 | 0,0 | 0,0 | 0,0 | 0,0 | 0,0 | 47,0 | 65,4 | 88,5 | 93,4 | 97,5 |
| Muster 4 | 0,0 | 0,0 | 0,0 | 0,0 | 0,0 | 58,8 | 82,4 | 91,7 | 93,7 | 99,2 |
| **Mittelwert** | **0,0** | **0,0** | **0,0** | **0,0** | **0,0** | **56,4** | **73,5** | **90,7** | **93,4** | **97,0** |
| StdAbw. | 0,0 | 0,0 | 0,0 | 0,0 | 0,0 | 6,7 | 7,2 | 1,5 | 0,2 | 2,1 |

**Vergleichsbeispiel 3**

Erste Beschichtung (Präpellets):

20 g Blanose 7LF = 20% bezogen auf die Pellets (Wasseraufnahme: ca. 50 % in 15 min bei pH 6,0 in Phosphatpuffer, Mucoadhäsivität bei pH 6,0 in Phosphatpuffer gemessen nach Hassan u. Gallo: $\eta_b$ = ca. 270 mPa.s) werden zusammen mit 1,1 g Aerosil 200 = 5,5% bezogen auf Blanose und 1,52 g Polysorbat 80 (33%ig) 2,5% bezogen auf Blanose in 727,4 g demineralisiertem Wasser unter Rühren mit einem Propellerrührer gelöst. Anschließend werden 139,4 mg Desmopressin acetat = 0,062% in der Formulierung in 50 g demineralisiertem Wasser gelöst und zur Blanose-Lösung unter Rühren gegeben.

200 g neutrale Kerne (Non Pareilles) 850 - 1000 μm werden im Mini-Wirbelschichtgerät (Mini-Glatt, Fa.Glatt, Binzen) vorgelegt und mit der Desmopressin - Blanose-Lösung beschichtet.

Sprühparameter:

Sprühdüse 0,5 mm
Sprührate 1,4 - 2,0 g/min
Sprühdruck 1 bar
Zuluftdruck 1,2 bar
Zulufttemperatur 45 - 47°C
Produkttemperatur 41 - 42°C
Sprühzeit: ca. 2 bis 6 h
Nachtrocknung im Mini Glatt 10 min bei 44°C
Trocknung über Nacht bei RT
Schichtdicke (REM): 10-12 μm,

**Vergleichsbeispiel 4**

Zweite Beschichtung (Pellets):

66,7 g (Meth)acrylatcopolymer-Dispersion (30-ige Dispersion, enthaltend ein Copolymer aus Methylacrylat/ Butylmethacrylat/Ethylacrylat/Methacrylsäure im Verhältnis 20/20/30/30, Röhm GmbH & Co. KG, Darmstadt) werden in einem 250 mL Becherglas vorgelegt. 2,4 g Polysorbat 80 (33%ig) = 40% bezogen auf GMS werden unter Rühren in 81 g demineralisiertes Wasser gelöst. Nach Zugabe von 2,0 g GMS = 10% bezogen auf Lacktrockensubstanz wird die Dispersion auf 80°C erhitzt. Die Dispersion wird nach Abkühlen auf 30°C mit dem Ultraturrax 10 min homogenisiert und anschließend unter Rühren zur Präparat 4154 D Dispersion gegeben. Nach 30 min Rühren werden damit 100 g der Desmopressin - Blanose beschichteten Pellets aus Beispiel 3 im MiniGlatt vorgelegt und mit der Dispersion beschichtet.

Sprühparameter:

Sprühdüse 0,5 mm
Sprührate 0,6 - 0,9 g/min
Sprühdruck 0,5 bar
Zuluftdruck 0,7 bar
Zulufttemperatur 35 - 37°C
Produkttemperatur 32 - 33°C
Nachtrocknung im Mini Glatt 10 min bei 40°C
Sprühzeit: ca. 1 bis 2 h
Trocknung über Nacht bei RT
Schichtdicke (REM): 40 - 45 μm,

Tabelle 4: Desmopressin-Freigabe der mit dem oben genannten Copolymer beschichteten Pellets, 2 h in 0.1 M HCl, 1 h in Phosphatpuffer pH 6,0; 100 rpm; Paddle; 37°C; n=4 (zweiter Überzug)

| Zeit [min] | 0 | 60 | 120 | 125 | 130 | 140 | 145 | 160 | 170 | 180 |
|---|---|---|---|---|---|---|---|---|---|---|
| Muster 1 | 0,0 | 0,0 | 0,0 | 0,0 | 0,0 | 24,8 | 47,4 | 93,0 | 98,0 | 102,4 |
| Muster 2 | 0,0 | 0,0 | 0,0 | 0,0 | 0,0 | 22,4 | 40,7 | 87,2 | 92,5 | 98,2 |
| Muster 3 | 0,0 | 0,0 | 0,0 | 0,0 | 0,0 | 20,8 | 42,5 | 88,4 | 95,6 | 101,5 |
| Muster4 | 0,0 | 0,0 | 0,0 | 0,0 | 0,0 | 25,1 | 41,9 | 88,9 | 98,8 | 97,9 |
| Mittelwert | **0,0** | **0,0** | **0,0** | **0,0** | **0,0** | **23,3** | **43,1** | **89,3** | **96,2** | **100,0** |

(fortgesetzt)

| Zeit [min] | 0 | 60 | 120 | 125 | 130 | 140 | 145 | 160 | 170 | 180 |
|---|---|---|---|---|---|---|---|---|---|---|
| StdAbw. | 0,0 | 0,0 | 0,0 | 0,0 | 0,0 | 2,0 | 2,9 | 2,5 | 2,8 | 2,3 |

**Beispiel 5**

Erste Beschichtung (Präpellets):

15 g Chitosan low MW (Fluka) = 10% bezogen auf die Pellets (Wasseraufnahme Chitosan: ca. 140 % in 15 min bei pH 5,5 in Acetatpuffer, Mucoadhäsivität bei pH 5,5 gemessen nach Hassan u. Gallo: eta-b = ca. 220 mPa.s) werden zusammen mit 0,825 g Aerosil 200 =5,5 % bezogen auf Chitosan in 1122 g demineralisiertem Wasser und 1,36 g Polysorbat 80 (33%ig) = 3% bezogen auf Chitosan unter Rühren mit einem Propellerrührer dispergiert. Anschließend wird das Chitosan durch Zugabe von 60 g Essigsäure unter weiterem Rühren innerhalb von 1 h gelöst. 104,6 mg Desmopressin acetat = 0,063% in der Formulierung in 50 g demin. Wasser gelöst und zur Chitosanlösung gegeben. 150 g non pareil pellets 850 - 1000 μm werden im MiniGlatt (Glatt, Binzen) vorgelegt und mit der Desmopressin - Chitosan lösung beschichtet.

Sprühparameter:

Sprühdüse 0,5 mm
Sprührate 0,8-2,5 g/min
Sprühdruck 1,5-1,8 bar
Zuluftdruck 1,1-1,2 bar
Zulufttemperatur 60-69°C
Produkttemperatur 59-62°C
Nachtrocknung im Mini Glatt 10 min bei 50°C
Sprühzeit 3-8h
Trocknung über Nacht bei RT
Schichtdicke (REM): 12 μm

Tabelle 5: Freigabe von Desmopressin in Phosphatpuffer pH 5,5; 100 min$^{-1}$; Paddle; 1 h; 100 rpm; 37°C; n=4; (erste Schicht)

| Zeit [min] | 0 | 5 | 10 | 15 | 20 | 25 | 30 | 45 | 60 |
|---|---|---|---|---|---|---|---|---|---|
| Muster 1 | 0,0 | 71,9 | 89,4 | 98,3 | 94,5 | 93,4 | 91,7 | 91,8 | 96,2 |
| Muster 2 | 0,0 | 68,6 | 91,5 | 105,3 | 99,3 | 92,9 | 100,3 | 104,0 | 98,6 |
| Muster 3 | 0,0 | 76,7 | 91,1 | 100,1 | 98,1 | 101,1 | 101,6 | 101,2 | 102,5 |
| Muster 4 | 0,0 | 70,7 | 92,6 | 100,3 | 94,8 | 99,5 | 99,3 | 97,1 | 94,6 |
| Mittelwert | 0,0 | 72,0 | 91,1 | 101,0 | 96,7 | 96,7 | 98,2 | 98,5 | 98,0 |
| SA | 0,0 | 3,4 | 1,4 | 3,0 | 2,4 | 4,2 | 4,4 | 5,3 | 3,4 |

**Beispiel 6**

Zweite Beschichtung (Pellets):

66,7 g EUDRAGIT® L30D-55 (30-%-ige Dispersion enthaltend ein (Meth)acrylatcopolymer aus 50 Gew.-% Methacrylsäure und 50 Gew.-% Ethylacrylat) werden mit 2 g Triethylcitrat (TEC) = 10% bezogen auf LTS (Lacktrockensubstanz) in einem 150 mL Becherglas gemischt. 0,73 g Polysorbat 80 (33%ig) = 40% bezogen auf GMS werden unter Rühren in 46 g demineralisiertes Wasser gelöst. Nach Zugabe von 0,6 g GMS = 3% bezogen auf LTS und demineralisiertem Wasser wird die Dispersion auf 80°C erhitzt. Die Dispersion wird nach Abkühlen auf 30°C mit dem Ultraturrax 10 min homogenisiert und anschließend unter Rühren zur EUDRAGIT® L30D-55 Dispersion gegeben. Nach 30 min Rühren werden damit 100 g der Desmopressin - Chitosan beschichteten Pellets aus Beispiel 5 im MiniGlatt vorgelegt und mit der EUDRAGIT® L30D-55 Dispersion beschichtet.

**Beispiel 7**: Sprühfähigkeit

Tabelle 7: Übersicht über Sprühfähigkeit (ja/nein) der Polymerdispersionen bzw. - Lösungen bei verschiedenen Konzentrationen.

| Mucoadhaesives Polymer | Konzentration | | |
|---|---|---|---|
| **pH 5.5** | **1.25%** | **2%** | **5%** |
| Blanose 7LF (Na-CMC) | n.a. | ja | ja |
| Chitosan (low Mw) | ja | nein | nein |
| Methocel A15 (Methylcellulose) | n.a. | ja | ja |
| **pH 7.2** | | | |
| (Meth)acrylatcopolymer* | n.a. | ja | ja |
| Methocel A15 (Methylcellulose) | n.a. | ja | ja |
| Blanose 7LF (Na-CMC) * | n.a. | ja | ja |
| *= (Meth)acrylatcopolymer aus 30 Gew.-% Methylmethacrylat und 70 Gew.-% Methacrylsäure | | | |

**Beispiel 8:** Formulierungsbeispiele für die gezielte Wirkstoffreigabe in verschiedenen Darmabschnitten (siehe Tabelle 8).

Partikelgröße nach dem Beschichten, fertige Formulierung bei Verwendung von Non pareil Pellets 850 -1000 als Trägermaterial $\mu$m → 900 - 1050 $\mu$m

**Tabelle 8: Rezepturen und Polymereigenschaften**

| pH Wert zugehöriger Darmabschnitt | Rezeptur Prozentuale Zusamensetzung in der Formulierung | Eigenschaften Mucoadhäsives Polymer | Wirkstoff * im mucoadh. Polymer ** in der Form. | Weitere Hilfsstoffe | Anwendungsgebiet, Therapie |
|---|---|---|---|---|---|
| 5,5 -Duodenum | 1. Schicht:<br>Neutral pellets 0,85-1mm 72,2%<br>Chitosan 7,2%<br>Essigsäure zur Auflösung Aerosil 200 0,4%<br>Polysorbat 80 0,22%<br>2. Schicht:<br>Eudragit® L30D-55 16,0%<br>TEC 1,6%<br>GMS 1,6%<br>Polysorbat 80 0,64% | Chitosan (Na-CMC)<br>$\eta_b$= *** = ca. 220 mPa.s<br><br>Wasseraufn. = ca. 140% in 15 min | *Desmopressin, 0,70%<br><br>**Desmopressin, 0,050% | | Diabetes insipidus |
| 6,0 -Jejunum | 1. Schicht:<br>Neutral pellets 0,85-1mm 73,2%<br>Blanose 7LF 7,3%<br>Aerosil 200 0,4%<br>Polysorbat 80 0,18%<br>2. Schicht:<br>Copolymer Beispiel 4 16,3%<br>GMS 1,6%<br>Polysorbat 80 0,65% | Blanose 7LF (Na-CMC)<br>Eta b* = ca. 270 mPa.s<br><br>Wasseraufn. = ca. 50 % in 15 min | *Insulin, 4,75 %<br><br>** Insulin, 0,35% | **Polyethylen-660-12-Hydroxy-Stearat<br><br>(Solutol® HS15) bis 2%<br>**Na-Glycocholate bis 1 %<br>**Aprotinin bis 1% | Diabetes mellitus (Basis Therapie) |

| pH Wert zugehöriger Darmabschnitt | Rezeptur Prozentuale Zusamensetzung in der Formulierung | Eigenschaften Mucoadhäsives Polymer | Wirkstoff * im mucoadh. Polymer ** in der Form. | Weitere Hilfsstoffe | Anwendungsgebiet, Therapie |
|---|---|---|---|---|---|
| 6,0 -Jejunum | 1. Schicht:<br>Neutral pellets 0,85-1mm 71,9%<br>Blanose 7LF 7,2%<br>Aerosil 200 0,4%<br>Polysorbat 80 0,18%<br>2. Schicht:<br>Copolymer Beispiel 4 16,3%<br>GMS 1,6%<br>Polysorbat 80 0,65% | Blanose 7LF (Na-CMC) Eta b* = ca. 270 mPa.s<br><br>Wasseraufn. = ca. 50 % in 15 min | *Cetrorelix, 24%<br><br>**Cetrorelix, 1,73%<br><br>u.andere LHRH Antagonisten | Polyoxyethylene-polyoxypropylene glycol<br><br>(Pluronic® F68) bis 2 % | Mamma-karzinom Prostata-karzinom |
| 7,2 -Ileum -Colon descendens | 1. Schicht:<br>Neutral pellets 0,85-1mm 72,8%<br>Blanose 7LF 7,3%<br>Aerosil 200 0,46%<br>GMS 0,22%<br>Polysorbat 80 0,087%<br>2. Schicht:<br>EUDRAGIT® FS30D 16,2%<br>TEC 0,81%<br>GMS 1,46%<br>Polysorbat 80 0,58% | Blanose 7LF (Na-CMC) Eta b = ca. 250 mPa.s<br><br>Wasseraufn. = ca. 52% in 15 min | *Desmopressin, 0,95%<br><br>**Desmopressin, 0,069% | | Enuresis noctuma |

EP 1 643 977 B1

| pH Wert zugehöriger Darmabschnitt | Rezeptur Prozentuale Zusamensetzung in der Formulierung | Eigenschaften Mucoadhäsives Polymer | Wirkstoff * im mucoadh. Polymer ** in der Form. | Weitere Hilfsstoffe | Anwendungsgebiet, Therapie |
|---|---|---|---|---|---|
| 7,2 -Ileum -Colon descendens | 1. Schicht:<br>Neutral pellets 0,85-1 mm 63,5%<br>Blanose 7LF 12,7%<br>Aerosil 200 0,79%<br>GMS 0,38%<br>Polysorbat 80 0,152%<br>2. Schicht:<br>EUDRAGIT® FS30D 16,2%<br>TEC 0,81%<br>GMS 1,46%<br>Polysorbat 80 0,58% | Blanose 7LF (Na-CMC) Eta b = ca. 250 mPa.s<br><br>Wasseraufn. = ca. 52% in 15 min | *Ciclosporin A, 27,2%<br><br>**Ciclosporin A, 3,45% | **Polyethylen-660-12-Hydroxy-Stearat<br><br>(Solutol® HS15) bis 2% | Immun-suppresivum |
| ***Messung der Mucoadhäsiven Eigenschaft nach Hassan und Gallo | | | | | |

EP 1 643 977 B1

**Beispiele für Ausführungformen mit wirkstoffhaltiger lipophiler Matrix**

**1. Beispiel: Ausführung einer Formulierung für eine schwerlösliche Proteine**

(Erythropoetin alfa; Löslichkeit in Wasser nach DAB 10 mindestens 500 Teile Wasser für 1 Teil Wirkstoff; entspr. 2 g / l).

### a) Vorbereitung der lipophilen Phase.

100 g Inwitor 312 (Schmelztemperatur 55-60°C) werden in einem Wasserbad bei 65°C geschmolzen und 50 g Inwitor 308 (Schmelztemperatur 30-34 °C) werden langsam in die Schmelze eingerührt. Das Wasserbad wird bis auf 50°C heruntergekühlt und 7,5 g Tocopherol acetat, 2 und 3,5 g Na-Glycocholat unter Rühren zugegeben. Die Temperatur des Bades kann somit weitere 5°C gesenkt werden ohne dass das Fett wieder erstarrt. Die resultierende lipophile Matrix hat somit eine Schmelztemperatur von 38-41 °C und eine aus den einzelnen Komponenten errechnete Löslichkeit in Wasser nach DAB 10 von mindestens 400 Teilen Wasser für 1 Teil Lipohile Matrix; entspr. 2,5 g/l). Zu dieser Lösung werden 330 mg Erythropoetin alfa (ca. 40 Mill I.E.) unter Rühren zugegeben.

### b) Herstellung einer Emulsion

750 ml destilliertes Wasser werden zunächst bei 45 °C erhitzt und 15 g Na-Caprinat als Emulgator (2 %) zugegeben. Diese Lösung wird dann mit Zugabe von Zitronensäure auf einem pH-Werte von ca. 7 justiert. Danach 1,5 g Sojabohnen-Trypsin inhibitor (Serin-peptidase Inhibitor) und 1,5 g Bacitracin (Aminopeptidase Inhibitor) werden in dieser Lösung unter Rühren zugegeben. Zu dieser Lösung wird dann unter energischem Rühren die lipophile Phase emulgiert. Der Emulsions-Prozess kann beendet werden, wenn keine lipophile

Tröpfchen nach mikroskopischer Untersuchung größer als 50-60 $\mu$m zu erkennen sind.

### c) Herstellung von muco-adhesiven Kernen

In einem GPCG1 mit Rotor Einsatz werden 350 g Na-Alginat Pulver, 145 mikrokristalline Cellulose und 5 g Zitronensäure dazu gemischt. Die Emulsion beschrieben in b) wird als Bindemittel im Rotoagglomerationsprozess mit einer Sprührate von ca. 90 g/min gesprüht.
Der Rotor wird bei 1700-1800 UPM, die Zuluft auf 42 m$^3$ / Stunde und die Temperatur der Luft auf 30 °C eingestellt. Unter diesen Bedingungen können Muco-Adhesive Kerne zwischen 250 und 600 $\mu$m mit einer Ausbeute bis zu 80 % produziert werden.
Eine therapeutische Dosierung von 240 $\mu$g ist in 0,5 g Pelletkernen enthalten.

### d) Herstellung von überzogenen Pellets

Die Pelletkerne aus c) werden mittels gängiger Wirbelschichtverfahren mit EUDRAGIT® FS 30D überzogen. Der Polymerauftrag beträgt 40 Gew.-% bezogen auf das Kerngewicht. Die Dispersion/Suspension zum Überziehen besteht aus:

| | |
|---|---|
| EUDRAGIT® FS 30 D | 44,65 % |
| Triethylcitrat | 0,67 % |
| Polysorbat 80 | 0,26 % |
| Glycerinmonostearat | 0,67 % |
| Wasser | 53,75 % |

Die somit erhaltenen Pellets können in einer Tablette mit üblichen pharmazeutischen Verfahren und Hilfsstoffen verpresst oder in Kapseln gefüllt werden.

**2. Beispiel: Ausführung einer Formulierung für schwerlösliche Peptide**

(Cetrorelix actetat; Löslichkeit in Wasser nach DAB 10 mindestens 1000 Teile Wasser für 1 Teil Wirkstoff; entspr. 1 g/l).

**a) Vorbereitung der lipophilen Phase**

13 g Imwitor 312 (Schmelztemperatur 55 - 60°C) werden mit 4 g Poloxamer 407 (Lutrol F127, Schmelztemperatur 50-55 °C) bei 65°C im Wasserbad geschmolzen. Anschließend werden 1 g Caprylsäure, 1 g Na-Caprylat und 1 g Tocopherolacetat unter Rühren zugesetzt. Die resultierende lipophile Matrix hat somit eine Schmelztemperatur von 40-48 °C und eine aus den einzelnen Komponenten errechnete Löslichkeit in Wasser nach DAB 10 mindestens 700 Teile Wasser für 1 Teil lipophile Matrix (entspr. 1,5 g/l). Nach Abkühlen der Lösung auf 45°C werden 3,0 g Cetroreiix acetat unter schnellem Rühren in die lipophile Phase eingerührt und abgekühlt.

**b) Herstellung einer Emulsion**

Die erhaltene Dispersion aus a) wird mit dem Ultraturrax (20000 U/min) mit der Chitosan citrat Dispersion aus b) unter weiterer Abkühlung mit dem Eisbad bis auf 10°C mind. 10 min dispergiert. Der Emulgier-Prozess kann beendet werden, wenn keine lipophile Tröpfchen nach mikroskopische Untersuchung größer als 50-60 $\mu$m zu erkennen sind.

**c) Herstellung von mucoadhaesiven Kernen**

20 g Chitosan werden in 1000 g Wasser dispergiert und anschließend unter sehr schnellem Rühren 20 g Citronensäure. In die erhaltene klare gelbliche viskose Lösung werden unter schnellem Rühren 2 g Na-Dodecanat zugesetzt und 1 h weitergerührt.

Die Emulsion aus b) wird mit dem GPCG1 (Glatt) mit einer Sprührate von 10 - 12 g/min/kg auf 250 g Neutral-Pellets 400 - 600 $\mu$m bei einer Zulufttemperatur von 30°C und aufgesprüht. Die Zuluft wird hierbei auf 45 - 50m$^3$/h eingestellt. Die Ausbeute liegt hierbei bei 90%.

**d) Herstellung von überzogenen Pellets**

Die so erhaltenen Pellets werden mittels gängiger Wirbelschichtverfahren mit EUDRAGIT® L12.5 überzogen. Der Polymerauftrag beträgt 40 Gew.-% bezogen auf das Kerngewicht. Die Suspension zum Überziehen besteht aus:

| | |
|---|---|
| EUDRAGIT® L12.5 | 53,3% |
| Triethylcitrat | 1,33% |
| Isopropanol | 38,3% |
| Talkum | 2,0 % |
| Wasser | 5,0% |

Die somit erhaltenen Pellets können in einer Tablette mit üblichen pharmazeutischen Verfahren und Hilfsstoffen verpresst oder in Kapseln gefüllt werden.

**3. Beispiel: Ausführung einer Formulierung für ein schwerlösliches Protein**

(beta-Interferon human; Löslichkeit in Wasser nach DAB 10 mindestens 600 Teile Wasser für 1 Teil Wirkstoff; entspr. 2 g/l)

**a) Vorbereitung der lipophilen Phase**

400 g Imwitor 312 (Schmelztemperatur 55 - 60°C) und 200 g Dynasan 114 (Schmelztemperatur 55 - 58°C) werden mit 30 g Tocopherolacetat bei 65°C geschmolzen und in einen Granulator (Bohle) gegeben. Dazu werden 20 g Na-Caprylat unter Rühren zugegeben. Die Mischung wird auf 45°C abgekühlt und darin 100 mg Interferon-beta gelöst. Die resultierende lipophile Matrix hat somit eine Schmelztemperatur von 39-46°C und eine aus den einzelnen Komponenten errechnete Löslichkeit in Wasser nach DAB 10 mindestens 840 Teilen Wasser für 1 Teil lipophile Matrix. Die lipophile Matrix wird unter Kühlung auf eine Teilchengröße unter 50 $\mu$m gemahlen.

**b) Herstellung einer Pufferlösung**

1 g Na-Citrat und 1 g Citronensäure werden in 500 g Wasser gelöst. Unter schnellem Rühren werden 0,5 g Na-Cholat und 100 mg Sojabohnen-TrypsinInhibitor zugegeben.

**c) Granulation**

Die gemahlene wirkstoffhaltige lipophile Matrix aus a) wird in einem Granulator mit 1500 g Blanose 7LF gemischt. Anschließend wird mit der wäßrigen Pufferlösung aus b) granuliert, so dass 0,2 bis 0,5 mm große Partikel entstehen, die auf einem Spheronizer ausgerundet werden. Die erhaltenen feuchten Kerne werden bei 30 bis 25 °C in einem Wirbelschichttrockner milde getrocknet.

**d) Herstellung von überzogenen Pellets**

Die so Kerne aus c) werden mittels gängiger Wirbelschichtverfahren mit EUDRAGIT® FS 30D überzogen. Der Polymerauftrag beträgt 40 Gew.-% bezogen auf das Kerngewicht. Die Dispersion/Suspension zum Überziehen besteht aus:

| | |
|---|---|
| EUDRAGIT® FS 30 D | 44,65 % |
| Triethylcitrat | 0,67 % |
| Polysorbat 80 | 0,26 % |
| Glycerinmonostearat | 0,67 % |
| Wasser | 53,75 % |

Die somit erhaltenen Pellets können zu einer Tablette mit üblichen pharmazeutischen Verfahren und Hilfsstoffen verpresst oder in Kapseln gefüllt werden.

**Patentansprüche**

1. Orale multipartikuläre Arzneiform, enthaltend Pellets mit einer Größe im Bereich von 50 bis 2500 $\mu$m, die im wesentlichen aufgebaut sind aus

    a) einer inneren Matrix-Schicht, enthaltend einen Wirkstoff, der ein Peptid oder ein Protein einschließlich deren Derivate oder Konjugate ist und in eine Matrix aus einem Polymeren mit mucoadhaesiver Wirkung eingebettet ist, wobei die Matrix optional weitere pharmazeutisch übliche Hilfsstoffe enthalten kann, und
    b) einem äußeren verfilmten Überzug, bestehend im wesentlichen aus einem anionischen Polymeren oder Copolymeren, das optional mit pharmazeutisch üblichen Hilfsstoffen, insbesondere Weichmachern formuliert sein kann,

    **dadurch gekennzeichnet, daß**
    die multipartikuläre Arzneiform so formuliert ist, daß die enthaltenen Pellets im pH-Bereich des Magens freigesetzt werden, der äußere Überzug durch die Wahl des anionischen Polymeren oder Copolymeren bzw. seiner Formulierung mit Hilfsstoffen und seiner Schichtdicke so eingestellt ist, daß sich dieser in pH-Bereichen von 4,0 bis 8,0 im Darm innerhalb 15 bis 60 min auflöst, so daß die wirkstoffhaltige, mucoadhaesive Matrix-Schicht frei wird, an die Darmmucosa binden und dort den Wirkstoff freisetzen kann, wobei das Polymere mit mucoadhaesiver Wirkung so gewählt ist, daß es in einem. Bereich +/- 0,5 pH-Einheiten bezogen auf den pH-Wert, bei dem sich der äußere Überzug aufzulösen beginnt, eine mucoadhaesive Wirkung von $\eta_b$= 150 bis 1000 mPa·s und eine Wasseraufnahme von 10 bis 750 % in 15 min aufweist und der Wirkstoffanteil der Matrixschicht maximal 40 Gew.-% des Gehaltes am Polymeren mit mucoadhaesiver Wirkung beträgt, wobei das Polymere mit mucoadhaesiver Wirkung ein Chitosan, ein (Meth)acrylatcopolymer, bestehend aus 20 - 40 Gew.-% Methylmethacrylat und 60 bis 80 Gew.-% Methacrylsäure, eine vernetzte und/oder unvernetzte Polyacrylsäure, ein Na-Alginat, und/oder ein Pektin ist

2. Arzneiform nach Anspruch 1, **dadurch gekennzeichnet, daß** der äußere verfilmte Überzug Celluloseglycolat (Duodcell®), Celluloseacetatphtalat (CAP, Cellulosi acetas, PhEur, Celluloseacetate-phtalate, NF, Aquateric®), Celluloseacetatsuccinat (CAS), Celluloseacetattrimeliat (CAT), Hydroxypropylmethylcellulosephtalat (HPMCP, HP50, HP55), Hydroxypropylmethylcelluloseacetatsuccinat (HPMCAS -LF, -MF, -HF), Polyvinylacetatphtalat (PVAP, Sureteric®), Vinylacetat-Vinylpyrolidon-Copolymer (PVAc, Kollidon® VA64), Vinylacetat:Crotonsäure-Copolymer 9:1 (VAC:CRA, Kollicoat® VAC) und/oder Shelllack ist.

3. Arzneiform nach Anspruch 1, **dadurch gekennzeichnet, daß** der äußere verfilmte Überzug aus einem (Meth) acrylat-Copolymeren mit einem Gehalt an Monomeren mit anionischen Gruppen von 5 bis 60 Gew.-% besteht.

4. Arzneiform nach einem oder mehreren der Ansprüche 1 bis 3, **dadurch gekennzeichnet, daß** die Schichtdicke des äußeren Überzuges im Bereich von 20 bis 200 $\mu$m liegt.

5. Arzneiform nach einem oder mehreren der Ansprüche 1 bis 4 , **dadurch gekennzeichnet, daß** die innere Matrix eine $C_6$- bis $C_{20}$-Fettsäure und/oder einen $C_6$- bis $C_{20}$-Alkohol einschließlich deren Salze, Ether-, Ester- oder Amid-Derivaten und/oder ein Lipid und/oder ein Phospholipid und/oder ein lipidlösliches Vitamin und/oder einen Proteaseinhibitor und/oder einen Penetrationsförderer enthält.

6. Arzneiform nach Anspruch 5, **dadurch gekennzeichnet, daß** die innere Matrix als Polymer mit mucoadhaesiver Wirkung ein Chitosan enthält, das zusammen mit einer Säure oder einem Puffersystem eingesetzt wird, die bzw. das sich in der Matrix oder in oder auf einem Kern, auf dem die Matrix aufgetragen wird, befindet.

7. Arzneiform nach Anspruch 6, **dadurch gekennzeichnet, daß** die innere Matrix-Schicht Chitosan enthält und mittels einer Säure oder einem Puffersystem auf pH 5,0 bis 5,5 eingestellt wird und mit einem aüßeren verfilmten Überzug kombiniert wird, der sich im Bereich von pH 6,0 bis 8,0 aufzulösen beginnt.

8. Arzneiform nach einem oder mehreren der Ansprüche 1 bis 7, **dadurch gekennzeichnet, daß** der Wirkstoff ein Protein oder Peptid mit einem mittleren Molekulargewicht $M_w$ von kleiner 3.000 ist.

9. Arzneiform nach Anspruch 8, **dadurch gekennzeichnet, daß** der Wirkstoff Abarelix, Angiotensin II, Anidulafungin, Antide, Argipressin, Azalin und Azalin B, Bombesin-Antagonist, Bradykinin, Buserelin, Cetrorelix, Ciclosporin A, Desmopressin, Detirelix, Enkephaline (Leu-, Met-) Ganirelix, Gonadorelin, Goserelin, Growthhormone-Secretagogue, Micafungin, Nafarelin, Leuprolide, Leuprorelin, Octreotid, Orntide, Oxytocin, Ramorelix, Sekretin, Somatotropin, Terlipressin, Tetracosactid, Teverelix, Triptorelin, Thyroliberin, Thyrotropin oder Vasopressin ist.

10. Arzneiform nach Anspruch 8 oder 9, **dadurch gekennzeichnet, daß** die Matrix-Schicht zusätzlich Matrix eine $C_6$- bis $C_{20}$-Fettsäure und/oder einen $C_6$- bis $C_{20}$-Alkohol einschließlich deren Salze, Ether-, Ester- oder Amid-Derivaten und/oder ein Lipid und/oder ein Phospholipid und/oder ein lipidlösliches Vitamin enthält.

11. Arzneiform nach einem oder mehreren der Ansprüche 1 bis 7, daß der Wirkstoff einen Protein oder Peptid mit einem mittleren Molekulargewicht $M_w$ von 3.000 bis 10.000 ist.

12. Arzneiform nach Anspruch 11, **dadurch gekennzeichnet, daß** der Wirkstoff Calcitonin, Corticotropin, Endorphine, Epithelial growth factor, Glucagon, Insulin, Novolin, Parathyroid Hormon, Relaxin, Pro-Somatostatin oder Salmon Secretin ist.

13. Arzneiform nach Anspruch 11 oder 12, **dadurch gekennzeichnet, daß** die Matrix-Schicht einen $C_6$- bis $C_{20}$-Alkohol einschließlich deren Salze, Ether-, Ester- oder Amid-Derivaten und/oder ein Lipid und/oder ein Phospholipid und/ oder ein lipidlösliches Vitamin und/oder einen Proteaseinhibitor enthält.

14. Arzneiform nach einem oder mehreren der Ansprüche 1 bis 8, **dadurch gekennzeichnet, daß** der Wirkstoff einen Protein oder Peptid mit einem mittleren Molekulargewicht $M_w$ von mehr als 10.000 ist.

15. Arzneiform nach Anspruch 14, **dadurch gekennzeichnet, daß** der Wirkstoff Interferon (alpha, beta, gamma), Interleukine (IL1, IL2), Somatotropin, Erytropoietin, Tumornekrosefaktor (TNF alpha, beta), Relaxin, Endorphin, Dornase alpha, Folikel stimulierendes Hormon (FSH), Human Chorion Gonadotropin (HCG), Human Growth Hormone Release factor (hGRF), Luteinisierendes Hormon (LH) oder Epidermal Growth Factor ist.

16. Arzneiform nach Anspruch 14 oder 15, **dadurch gekennzeichnet, daß** die Matrix-Schicht eine $C_6$- bis $C_{20}$-Fettsäure und/oder einen $C_6$- bis $C_{20}$-Alkohol einschließlich deren Salze, Ether-, Ester- oder Amid-Derivaten und/oder ein Lipid und/oder ein Phospholipid und/oder ein lipidlösliches Vitamin und/oder einen Proteaseinhibitor und/oder einen Penetrationsförderer enthält.

17. Arzneiform nach einem oder mehreren der Ansprüche 1 bis 16, **dadurch gekennzeichnet, daß** zwischen der wirkstoffhaltigen Matrix-Schicht und der äußeren verfilmten Überzugsschicht eine Trennschicht aufgebracht ist.

18. Verfahren zur Herstellung einer multipartikulären Arzneiform nach einem oder mehreren der Ansprüche 1 bis 17, indem man

    a) eine innere Matrix-Schicht, enthaltend einen Wirkstoff, der ein Peptid oder ein Protein ist, und ein Polymer mit mucoadhaesiver Wirkung und gegebenenfalls weitere pharmazeutisch übliche Hilfsstoffe mittels Sprühauftrag auf einen Kern oder durch Rotagglomeration, Ausfällen oder Sprühverfahren ohne einen Kern herstellt und anschließend
    b) einen äußeren verfilmten Überzug, bestehend im wesentlichen aus einem anionischen Polymer oder Copolymer, das optional mit pharmazeutisch üblichen Hilfsstoffen, insbesondere Weichmachern, formuliert sein kann, mittels Sprühauftag aufbringt, so daß wirkstoffhaltige, umhüllte Pellets erhalten werden, und man
    c) die erhaltenen Pellets mittels pharmazeutisch üblicher Hilfsstoffe und in an sich bekannter Weise zu einer multipartikulären Arzneiform, insbesondere zu pellethaltigen Tabletten, Minitabletten, Kapseln, Sachets oder Trockensäften verarbeitet, die so formuliert sind, daß die enthaltenen Pellets im pH-Bereich des Magens freigesetzt werden.

19. Arzneiform nach einem oder mehreren der Ansprüche 1 bis 17, **dadurch gekennzeichnet, daß** der Wirkstoff in eine lipophile Matrix eingebettet ist, die einen Schmelzpunkt oberhalb von 37 °C aufweist, und die wirkstoffhaltige lipophile Matrix in die Matrix aus dem Polymeren mit mucoadhaesiver Wirkung eingebettet ist.

20. Arzneiform nach Anspruch 19, **dadurch gekennzeichnet, daß** sich der Wirkstoff und die Substanz oder die Substanzen, die die lipophile Matrix bilden, sich in ihrer Löslichkeit in Wasser nach DAB 10 und nicht mehr als +/- 50 % unterscheiden und/oder in ihrem Verteilungskoeffizienten nach Anhang V zu RL 67/548/EWG, A.8, nach nicht mehr als +/- 60 % unterscheiden und/oder in ihrem HLB-Wert gemessen nach Marszall nicht mehr als +/- 80 % unterscheiden.

21. Arzneiform nach Anspruch 19 oder 20, **dadurch gekennzeichnet, daß** ein Wirkstoff enthalten ist, der eine Löslichkeit in Wasser nach DAB 10 von mindestens 30 Volumenteilen Wasser für einen Gewichtsteil Wirkstoff aufweist.

22. Arzneiform nach Anspruch 21, **dadurch gekennzeichnet, daß** der Wirkstoff aus der Gruppe der Peptidantibiotika, Immunsupressiva, LHRH-Antagonisten, Immunomodulatoren ausgewählt ist.

23. Arzneiform nach Anspruch 21 oder 22, **dadurch gekennzeichnet, daß** der Wirkstoff Abarelix, Angiotensin II, Anidulafungin, Antide, Argipressin, Azalin und Azalin B, Bombesin-Antagonist, Bradykinin, Buserelin, Calcitonin, Cetrorelix, Ciclosporin, Ciclosporin A, Desmopressin, Detirelix, Erytropoietin, Enkephaline (Leu-, Met-) Ganirelix, Gonadorelin, Goserelin, Growthhormone-Secretagogue, Insulin, Interferon (alpha, beta, gamma), Interleukine (IL1, IL2), Micafungin, Nafarelin, Leuprolide, Leuprorelin, Octreotid, Orntide, Oxytocin, Parathyroid Hormon, Ramorelix, Sekretin, Somatotropin, Terlipressin, Tetracosactid, Teverelix, Triptorelin, Thyroliberin, Thyrotropin Tumornekrosefaktor (TNF alpha, beta),oder Vasopressin ist.

24. Arzneiform nach einem oder mehreren der Ansprüche 19 bis 23, **dadurch gekennzeichnet, daß** die Substanz oder die Substanzen, die die lipophile Matrix bilden, und das Polymer mit mucoadhaesiver Wirkung entweder die gleiche ionische Eigenschaft aufweisen oder dass im Falle entgegengesetzter ionischer Eigenschaften, das Polymer mit mucoadhaesiver Wirkung zu mindestens 50 % in neutralisierter Form vorliegt.

25. Arzneiform nach einem oder mehreren der Ansprüche 19 bis 24, **dadurch gekennzeichnet, daß** die lipophile Matrix zu 80 bis 100 Gew.-% aus einer Substanz mit einem HLB-Wert von 0 bis 15 oder einer Mischung von Substanzen mit einem gemittelten HLB-Wert von 0 bis 15 besteht und 0 bis 20 Gew.-% an pharmazeutisch üblichen Hilfsstoffen, insbesondere Stabilisatoren, Verdickungsmittel oder Adsorbentien, enthalten kann.

26. Arzneiform nach einem oder mehreren der Ansprüche 19 bis 25 **dadurch gekennzeichnet, daß** die Substanz oder die Substanzen, die die lipophile Matrix bilden, zu der Gruppe der Öle, Fette, Mono-, Di- oder Triglyceride, Fettsäuren, Fettalkohole, insbesondere $C_6$- bis $C_{20}$-Fettsäure und/oder einen $C_6$- bis $C_{20}$-Alkohol einschließlich deren Salze, Ether-, Ester- oder Amid-Derivaten, Phospholipide, Lecithine, Emulgatoren, Lipoide, lipidlösliches Vitamine oder Tenside gehören.

27. Arzneiform nach einem oder mehreren der Ansprüche 19 bis 25, **dadurch gekennzeichnet, daß** die lipophile Matrix eine der folgenden Lipidpräparationen enthält: (Imwitor 308) Glycerylmonocaprylate mit einem Monoesteranteil >80 %, (Imwitor 312) Glycerylmonolaurate mit enem Monoesteranteil > 90 %, (Imwitor 491) Glycerolmonosterate ($C_{16}$ + $C_{18}$) mit einem Monoesteranteil >90 %, (Imwitor 900 P) Glycerolmonosterat mit einem Monoesteranteil von 40 - 55% und einem $C_{18}$-Gehalt von 40 - 60%, (Imwitor 900 K) Glycerolmonosterat, mit einem Monoesteranteil von 40 - 55 % und einem $C_{18}$-Gehalt von 60 -80 %, (Imwitor 742) Mittelkettige $C_8$ und $C_{10}$ Glyceride mit einem Monoesteranteil von 45 - 55 %, (Imwitor 928) Partielle Glyceride gesättigter pflanzlicher $C_{10}$-$C_{18}$Fettsäuren mit einem Hauptanteil an $C_{12}$, und mit einem Monoesteranteil von 34-36%, $C_8$ and $C_{10}$-Glyceride, NaCaprylat oder NaCapriat.

28. Arzneiform nach einem oder mehreren der Ansprüche 19 bis 27 , **dadurch gekennzeichnet, daß** der Wirkstoff zu mindestens 10 % in der lipophilen Matrix löslich ist.

29. Arzneiform nach einem oder mehreren der Ansprüche 19 bis 28, **dadurch gekennzeichnet, daß** der Gehalt der wirkstoffhaltigen lipophilen Matrix an der inneren Matrix-Schicht a) 5 bis 50 Gew.-% beträgt.

30. Verfahren zur Herstellung einer multipartikulären Arzneiform nach einem oder mehreren der Ansprüche 19 bis 29, mit den Schritten

a) Erzeugen der wirkstoffhaltigen lipophilen Matrix durch Suspendieren und/oder Lösen des Wirkstoffs mit der oder den Substanzen, die die lipophile Matrix bilden und gegebenenfalls weiteren pharmazeutisch üblichen Hilfsstoffen durch intensives Mischen oder Schmelzen der Inhaltsstoffe
b) Erzeugen von Präpellets (Pelletkerne) mittels Sprühauftrag des mucoadhaesiven Polymers in Mischung mit der wirkstoffhaltigen lipophilen Matrix auf einen Kern oder durch Rotagglomeration, Ausfällen oder Sprühverfahren ohne einen Kern,
c) Erzeugen von Pellets durch Sprühauftrag eines Überzuges des anionischen Polymers oder Copolymers, das optional Beimengungen pharmazeutisch üblicher Hilfsstoffe, insbesondere Weichmachern und Trennmittel, enthalten kann, aus einer Dispersion oder organischer Lösung auf die Präpellets aus Schritt b),
d) Herstellung einer multipartikulären Arzneiform, durch Verfüllen oder Einarbeiten der Pellets aus Schritt c) in an sich bekannter Weise, gegebenenfalls unter Verwendung pharmazeutisch üblicher Hilfsstoffe, insbesondere durch Verarbeitung zu pellethaltigen Tabletten, Minitabletten, Kapseln, Sachets oder Trockensäften.

31. Verfahren zur Herstellung einer multipartikulären Arzneiform nach Anspruch 30, **dadurch gekennzeichnet, daß** die Schritte a) und b) wie folgt ausgeführt werden

a) Erzeugen der inneren Matrix-Schicht durch Herstellen Emulsion, Dispersion oder Lösung des Wirkstoffs mit der oder den Substanzen für die lipophilen Matrix, und gegebenenfalls weiteren pharmazeutisch üblichen Hilfsstoffen durch intensives Mischen der Inhaltsstoffe in Wasser und Erzeugung einer Öl in Wasser-Präparation mit einer mittleren Teilchengröße von nicht mehr als 60 $\mu$m,
b) Erzeugen von Präpellets mittels Sprühauftrag der Öl in Wasser-Präparation aus Schritt a) auf das mucoadhaesive Polymer, das gegebenenfalls Beimengungen weiterer pharmazeutisch üblicher Hilfsstoffe enthalten kann, wobei die Inhaltsstoffe in Form eines mikronisierten Pulvers vorliegen, durch Rotoagglomeration Extrusion oder Granulation.

## Claims

1. Oral multiparticulate pharmaceutical form comprising pellets having a size in the range from 50 to 2500 $\mu$m, which are substantially composed of

a) an inner matrix layer comprising an active substance which is a peptide or a protein, including derivatives or conjugates thereof, and is embedded in a matrix of a polymer having a mucoadhesive effect, where the matrix may optionally comprise further pharmaceutically usual excipients, and
b) an outer film coating consisting essentially of an anionic polymer or copolymer which may optionally be formulated with pharmaceutically usual excipients, especially plasticizers,

**characterized in that**
the multiparticulate pharmaceutical form is formulated so that the contained pellets are released in the pH range of the stomach, the outer coating is adjusted through the choice of the anionic polymer or copolymer or its formulation with excipients and its layer thickness such that the coating dissolves in pH ranges from 4.0 to 8.0 in the intestine within 15 to 60 min, so that the active substance-containing, mucoadhesive matrix layer is exposed, and can bind to the intestinal mucosa and release the active substance there, where the polymer having a mucoadhesive effect is chosen so that it exhibits a mucoadhesive effect of $\eta_b$ = 150 to 1000 mPa·s and a water uptake of from 10 to 750% in 15 min in a range of +/- 0.5 pH units relative to the pH at which the outer coating starts to dissolve, and the active substance content of the matrix layer is a maximum of 40% by weight of the content of polymer having a mucoadhesive effect the polymer having a mucoadhesive effect, being a chitosan, a (meth)acrylate copolymer consisting of 20-40% by weight methyl methacrylate and 60 to 80% by weight methacrylic acid, a crosslinked and/or uncrosslinked polyacrylic acid, an Na alginate, and/or a pectin.

2. Pharmaceutical form according to Claim 1, **characterized in that** the outer film coating is cellulose glycolate (Duodcell®), cellulose acetate phthalate (CAP, Cellulosi acetas, PhEur, cellulose acetate phthalates, NF, Aquaterie®), cellulose acetate succinate (CAS), cellulose acetate trimeliate (CAT), hydroxypropylmethylcellulose phthalate (HPMCP, HP50, HP55), hydroxypropylmethylcellulose acetate succinate (HPMCAS-LF, -MF, -HF), polyvinyl acetate phthalate (PVAP, Sureteric®) , vinyl acetate-vinylpyrrolidone copolymer (PVAc, Kollidon® VA64), vinyl acetate:crotonic acid 9:1 copolymer (VAC:CRA, Kollicoat® VAC) and/or shellack.

3. Pharmaceutical form according to Claim 1, **characterized in that** the outer film coating consists of a (meth)acrylate copolymer having a content of monomers having anionic groups of from 5 to 60% by weight.

4. Pharmaceutical form according to one or more of Claims 1 to 3, **characterized in that** the layer thickness of the outer coating is in the range from 20 to 200 $\mu$m.

5. Pharmaceutical form according to one or more of Claims 1 to 4, **characterized in that** the inner matrix comprises a $C_6$- to $C_{20}$-fatty acid and/or a $C_6$- to $C_{20}$-alcohol including their salts, ether, ester or amide derivatives and/or a lipid and/or a phospholipid and/or a lipid-soluble vitamin and/or a protease inhibitor and/or a penetration promoter.

6. Pharmaceutical form according to Claim 5, **characterized in that** the inner matrix comprises as polymer having a mucoadhesive effect a chitosan which is employed together with an acid or a buffer system, which is located in the matrix or in or on a core onto which the matrix is applied.

7. Pharmaceutical form according to Claim 6, **characterized in that** the inner matrix layer comprises chitosan and is adjusted to pH 5.0 to 5.5 by means of an acid or a buffer system, and is combined with an outer film coating which starts to dissolve in the range from pH 6.0 to 8.0.

8. Pharmaceutical form according to one or more of Claims 1 to 7, **characterized in that** the active substance is a protein or a peptide having an average molecular weight $M_w$ of less than 3000.

9. Pharmaceutical form according to Claim 8, **characterized in that** the active substance is abarelix, angiotensin II, anidulafungin, antide, argipressin, azaline and azaline B, bombesin antagonist, bradykinin, buserelin, cetrorelix, cyclosporin A, desmopressin, detirelix, encephalins (Leu-, Met-) ganirelix, gonadorelin, goserelin, growth hormone secretagogue, micafungin, nafarelin, leuprolide, leuprorelin, octreotide, orntide, oxytocin, ramorelix, secretin, somatotropin, terlipressin, tetracosactide, teverelix, triptorelin, thyroliberin, thyrotropin, vasopressin.

10. Pharmaceutical form according to Claim 8 or 9, **characterized in that** the matrix layer additionally matrix comprises a $C_6$- to $C_{20}$-fatty acid and/or a $C_6$- to $C_{20}$-alcohol including their salts, ether, ester or amide derivatives and/or a lipid and/or a phospholipid and/or a lipid-soluble vitamin.

11. Pharmaceutical form according to one or more of Claims 1 to 7, in that the active substance is a protein or peptide having an average molecular weight $M_w$ of from 3000 to 10 000.

12. Pharmaceutical form according to Claim 11, **characterized in that** the active substance is calcitonin, corticotrophin, endorphins, epithelial growth factor, glucagon, insulin, novolin, parathyroid hormone, relaxin, pro-somatostatin or salmon secretin.

13. Pharmaceutical form according to Claim 11 or 12, **characterized in that** the matrix layer comprises a $C_6$- to $C_{20}$-alcohol including their salts, ether, ester or amide derivatives and/or a lipid and/or a phospholipid and/or a lipid-soluble vitamin and/or a protease inhibitor.

**14.** Pharmaceutical form according to one or more of Claims 1 to 8, **characterized in that** the active substance is a protein or peptide having an average molecular weight $M_w$ of more than 10 000.

**15.** Pharmaceutical form according to Claim 14, **characterized in that** the active substance is interferon (alpha, beta, gamma), interleukins (IL1, IL2), somatotropin, erythropoietin, tumor necrosis factor (TNF alpha, beta), relaxin, endorphin, dornase alpha, follicle stimulating hormone (FSH), human chorion gonadotropin (HCG), human growth hormone release factor (hGRF), luteinizing hormone (LH) or epidermal growth factor.

**16.** Pharmaceutical form according to Claim 14 or 15, **characterized in that** the matrix layer comprises a $C_6$- to $C_{20}$-fatty acid and/or a $C_6$- to $C_{20}$-alcohol including their salts, ether, ester or amide derivatives and/or a lipid and/or a phospholipid and/or a lipid-soluble vitamin and/or a protease inhibitor and/or a penetration promoter.

**17.** Pharmaceutical form according to one or more of Claims 1 to 16, **characterized in that** a separating layer is applied between the active substance-containing matrix layer and the outer film coating layer.

**18.** Process for producing a multiparticulate pharmaceutical form according to one or more of Claims 1 to 17, by

a) producing an inner matrix layer comprising an active substance, which is a peptide or a protein, and a polymer having a mucoadhesive effect and, where appropriate, further pharmaceutically usual excipients by means of spray application onto a core or by rotagglomeration, precipitation or spray processes without a core, and subsequently
b) applying an outer film coating consisting essentially of an anionic polymer or copolymer, which may optionally be formulated with pharmaceutically usual excipients, especially plasticizers, by means of spray application so that active substance-containing, enveloped pellets are obtained, and
c) processing the resulting pellets by means of pharmaceutically usual excipients in a manner known per se to a multiparticulate pharmaceutical form, in particular to pellet-containing tablets, minitablets, capsules, sachets or reconstitutable powders, which are formulated so that the contained pellets are released in the pH range of the stomach.

**19.** Pharmaceutical form according to one or more of Claims 1 to 17, **characterized in that** the active substance is embedded in a lipophilic matrix which has a melting point above 37°C, and the active substance-containing lipophilic matrix is embedded in the matrix composed of the polymer having a mucoadhesive effect.

**20.** Pharmaceutical form according to Claim 19, **characterized in that** the active substance and the substance or substances forming the lipophilic matrix differ in their solubility in water according to DAB 10 and not more than +/- 50%, and/or differ in their partition coefficient according to annex V to directive 67/548/EEC, A.8 by not more than +/- 60%, and/or differ in their HLB measured by the method of Marszall not more +/- 80%.

**21.** Pharmaceutical form according to Claim 19 or 20, **characterized in that** an active substance which has a solubility in water according to DAB 10 of at least 30 parts by volume of water for one part by weight of active substance is present.

**22.** Pharmaceutical form according to Claim 21, **characterized in that** the active substance is selected from the group of peptide antibiotics, immunosuppressants, LHRH antagonists, immunomodulators.

**23.** Pharmaceutical form according to Claim 21 or 22, **characterized in that** the active substance is abarelix, angiotensin II, anidulafungin, antide, argipressin, azaline and azaline B, bombesin antagonist, bradykinin, buserelin, calcitonin, cetrorelix, cyclosporin, cyclosporin A, desmopressin, detirelix, erythropoietin, encephalins (Leu-, Met-) ganirelix, gonadorelin, goserelin, growth hormone secretagogue, insulin, interferon (alpha, beta, gamma), interleukins (IL1, IL2), micafungin, nafarelin, leuprolide, leuprorelin, octreotide, orntide, oxytocin, parathyroid hormone, ramorelix, secretin, somatotropin, terlipressin, tetracosactide, teverelix, triptorelin, thyroliberin, thyrotropin, tumor necrosis factor (TNF alpha, beta) or vasopressin.

**24.** Pharmaceutical form according to one or more of Claims 19 to 23, **characterized in that** the substance or substances forming the lipophilic matrix, and the polymer having a mucoadhesive effect either have the same ionic property or, in the event of opposed ionic properties, the polymer having a mucoadhesive effect is present in at least 50% neutralized form.

**25.** Pharmaceutical form according to one or more of Claims 19 to 24, **characterized in that** the lipophilic matrix consists of 80 to 100% by weight of a substance having an HLB of from 0 to 15 or of a mixture of substances having an average HLB of from 0 to 15, and may comprise from 0 to 20% by weight of pharmaceutically usual excipients, especially stabilizers, thickeners or adsorbents.

**26.** Pharmaceutical form according to one or more of Claims 19 to 25 **characterized in that** the substance or the substances forming the lipophilic matrix belong to the group of oils, fats, mono-, di- or triglycerides, fatty acids, fatty alcohols, especially $C_6$ to $C_{20}$-fatty acid and/or a $C_6$- to $C_{20}$-alcohol including their salts, ether, ester or amide derivatives, phospholipids, lecithins, emulsifiers, lipoids, lipid-soluble vitamins or surfactants.

**27.** Pharmaceutical form according to one or more of Claims 19 to 25, **characterized in that** the lipophilic matrix comprises one of the following lipid preparations: (Imwitor 308) glyceryl monocaprylates having a monoester content of > 80%, (Imwitor 312) glyceryl monolaurates having a monoester content of > 90%, (Imwitor 491) glycerol monostearates ($C_{16}$ + $C_{18}$) having a monoester content of > 90%, (Imwitor 900 P) glycerol monostearate having a monoester content of 40-55% and a $C_{18}$ content of 40-60%, (Imwitor 900 K) glycerol monostearate, having a monoester content of 40-55% and a $C_{18}$ content of 60-80%, (Imwitor 742) medium chain-length $C_8$ and $C_{10}$ glycerides having a monoester content of 45-55%, (Imwitor 928) partial glycerides of saturated vegetable $C_{10}$-$C_{18}$ fatty acids having a main content of $C_{12}$, and having a monoester content of 34-36%, $C_8$ and $C_{10}$ glycerides, Na caprylate or Na capriate.

**28.** Pharmaceutical form according to one or more of Claims 19 to 27, **characterized in that** the active substance is at least 10% soluble in the lipophilic matrix.

**29.** Pharmaceutical form according to one or more of Claims 19 to 28, **characterized in that** the content of active substance-containing lipophilic matrix in the inner matrix layer a) is from 5 to 50% by weight.

**30.** Process for producing a multiparticulate pharmaceutical form according to one or more of Claims 19 to 29, with the steps

> a) production of the active substance-containing lipophilic matrix by suspending and/or dissolving the active substance with the substance(s) which form the lipophilic matrix and, where appropriate, further pharmaceutically usual excipients by vigorously mixing or melting the ingredients,
> b) production of pre-pellets (pellet cores) by spray application of the mucoadhesive polymer mixed with the active substance-containing lipophilic matrix onto a core or by rotagglomeration, precipitation or spray processes without a core,
> c) production of pellets by spray application of a coating of the anionic polymer or copolymer, which may optionally comprise admixtures of pharmaceutically usual excipients, especially plasticizers and release agents, from a dispersion or organic solution onto the pre-pellets from step b),
> d) production of a multiparticulate pharmaceutical form by filling or incorporating the pellets from step c) in a manner known per se, where appropriate with use of pharmaceutically usual excipients, in particular by processing to pellet-containing tablets, minitablets, capsules, sachets or reconstitutable powders.

**31.** Process for producing a multiparticulate pharmaceutical form according to Claim 30, **characterized in that** steps a) and b) are carried out as follows

> a) production of the inner matrix layer by preparing an emulsion, dispersion or solution of the active substance with the substance(s) for the lipophilic matrix, and where appropriate further pharmaceutically usual excipients by vigorously mixing the ingredients in water and producing an oil-in-water preparation having an average particle size of not more than 60 $\mu$m,
> b) production of pre-pellets by spray application of the oil-in-water preparation from step a) onto the mucoadhesive polymer which may optionally comprise admixtures of further pharmaceutically usual excipients, where the ingredients are in the form of a micronized powder, by rotagglomeration, extrusion or granulation.

**Revendications**

**1.** Forme galénique multiparticulaire orale contenant des granulés présentant une taille de l'ordre de 50 à 2500 $\mu$m, constitués essentiellement :

a) d'une couche de matrice interne contenant un principe actif qui est un peptide ou une protéine, y compris leurs dérivés ou conjugués et est intégré dans une matrice constituée d'un polymère à effet muco-adhésif, la matrice pouvant contenir éventuellement d'autres adjuvants pharmaceutiques habituels, et

b) d'un revêtement pelliculé externe constitué essentiellement d'un polymère ou copolymère anionique qui peut être formulé éventuellement avec des adjuvants pharmaceutiques habituels, en particulier des plastifiants,

**caractérisée en ce que** :

la forme galénique multiparticulaire est formulée de telle sorte que les granulés contenus soient libérés dans une plage de pH de l'estomac, que le revêtement externe soit ajusté en choisissant le polymère ou copolymère anionique ou sa formulation avec des adjuvants et son épaisseur de couche de telle sorte que celui-ci se dissolve dans une plage de pH de 4,0 à 8,0 dans l'intestin, en l'espace de 15 à 60 minutes de sorte que la couche de matrice muco-adhésive contenant le principe actif soit libérée, puisse se lier à la muqueuse intestinale et y libérer le principe actif, le polymère présentant un effet muco-adhésif étant choisi de telle sorte qu'il présente, dans une plage de pH de +/- 0,5 par rapport à la valeur de pH à laquelle le revêtement externe commence à se dissoudre, un effet muco-adhésif de $\eta b = 150$ à $1000$ mPa et une absorption d'eau de 10 à 750 % en 15 min et que la proportion de principe actif de la couche de matrice soit au maximum de 40 % en poids du contenu du polymère présentant un effet muco-adhésif, le polymère à effet muco-adhésif étant un chitosan, un copolymère de (méth)acrylate, constitué de 20 à 40 % en poids de méthylméthacrylate et de 60 à 80 % en poids d'acide méthacrylique, un acide polyacrylique réticulé et/ou non réticulé, un alginate de sodium et/ou une pectine.

**2.** Forme galénique selon la revendication 1, **caractérisée en ce que** le revêtement pelliculé externe est le glycolate de cellulose (Duodcell®), l'acétate-phtalate de cellulose (CAP, Cellulosi acetas, Ph. Eur., l'acétatephtalate de cellulose, NF, Aquateric®), l'acétate-succinate de cellulose (CAS), l'acétate trimmélitate de cellulose (CAT), l'hydroxypropylméthylcellulose-phtalate (HPMCP, HP50, HP55), l'hydroxypropylméthylcellulose-acétate-succinate (HPMCAS-LF, -MF, -HF), l'acétate-phtalate de polyvinyle (PVAP, Sureteric®), le copolymère d'acétate de vinyle

- vinylpyrrolidone (PVAc, Kollidon® VA64), le copolymère acétate de vinyle - acide crotonique 9:1 (VAC:CRA, Kollicoat® VAC) et/ou le shellac.

**3.** Forme galénique selon la revendication 1, **caractérisée en ce que** le revêtement pelliculé externe est constitué d'un copolymère de (méth)acrylate présentant une teneur en monomère comportant des groupes anioniques de 5 à 60 % en poids.

**4.** Forme galénique selon une ou plusieurs des revendications 1 à 3, **caractérisée en ce que** l'épaisseur de couche du revêtement externe se situe dans une plage de 20 à 200 $\mu$m.

**5.** Forme galénique selon une ou plusieurs des revendications 1 à 4, **caractérisée en ce que** la matrice interne contient un acide gras en $C_6$ à $C_{20}$ et/ou un alcool en $C_6$ à $C_{20}$, y compris leurs sels, leurs dérivés d'éther, ester ou amide et/ou un lipide et/ou un phospholipide et/ou une vitamine liposoluble et/ou un inhibiteur de la protéase et/ou un agent de renforcement de la pénétration.

**6.** Forme galénique selon la revendication 5, **caractérisée en ce que** la matrice interne contient comme polymère à effet muco-adhésif, un chitosan qui est utilisé conjointement avec un acide ou un système de tampon qui se trouve dans la matrice ou dans ou sur un noyau sur lequel la matrice est appliquée.

**7.** Forme galénique selon la revendication 6, **caractérisée en ce que** la couche de matrice interne contient du chitosan et est ajustée à pH 5,0 à 5,5 avec un acide ou un système de tampon et est combinée à un revêtement pelliculé externe qui commence à se dissoudre dans une plage de pH 6,0 à 8,0.

**8.** Forme galénique selon une ou plusieurs des revendications 1 à 7, **caractérisée en ce que** le principe actif est une protéine ou un peptide présentant un poids moléculaire moyen $M_w$ inférieur à 3000.

**9.** Forme galénique selon la revendication 8, **caractérisée en ce que** le principe actif est l'abarelix, l'angiotensine II, l'anidulafungine, l'antide, l'argipressine, l'azaline et l'azaline B, l'antagoniste de la bombésine, la bradykinine, la buséréline, le cetrorelix, la ciclosporine A, la desmopressine, le detirelix, l'encéphaline (Leu-, Met-), le ganirelix, la gonadoreline, la gosereline, l'hormone de croissance sécrétagogue, la micafungine, la nafaréline, le leuprolide, la leuproréline, l'octréotide, l'orntide, l'oxytocine, le ramorelix, la sécrétine, la somatotropine, la terlipressine, le tétracosactide, le teverelix, la triptoréline, la thyrolibérine, la thyrotropine ou la vasopressine.

10. Forme galénique selon la revendication 8 ou 9, **caractérisée en ce que** la couche de matrice contient en outre une matrice contenant un acide gras en $C_6$ à $C_{20}$ et/ou un alcool en $C_6$ à $C_{20}$, y compris leurs sels, dérivés d'éther, ester ou amide et/ou un lipide et/ou un phospholipide et/ou une vitamine liposoluble.

11. Forme galénique selon l'une quelconque des revendications 1 à 7, **caractérisée en ce que** le principe actif est une protéine ou un peptide présentant un poids moléculaire moyen $M_w$ de 3000 à 10 000.

12. Forme galénique selon la revendication 11, **caractérisée en ce que** le principe actif est la calcitonine, la corticotropine, l'endorphine, le facteur de croissance épithéliale, le glucagon, l'insuline, la novoline, l'hormone parathyroïdienne, la relaxine, la pro-somatostatine ou la sécrétine de saumon.

13. Forme galénique selon la revendication 11 ou 12, **caractérisée en ce que** la couche de matrice contient un alcool en $C_6$ à $C_{20}$, y compris ses sels, dérivés d'éther, ester ou amide et/ou un lipide et/ou un phospholipide et/ou une vitamine liposoluble et/ou un inhibiteur de la protéase.

14. Forme galénique selon une ou plusieurs des revendications 1 à 8, **caractérisée en ce que** le principe actif est une protéine ou un peptide présentant un poids moléculaire moyen $M_w$ supérieur à 10 000.

15. Forme galénique selon la revendication 14, **caractérisée en ce que** le principe actif est un interféron (alpha, bêta, gamma), une interleukine (IL1, IL2), une somatotropine, une érythropoïétine, un facteur de nécrose tumorale (TNF alpha, bêta), une relaxine, une endorphine, une dornase alpha, une hormone de stimulation foliculaire (FSH), une gonadotropine chorionique humaine (HCG), un facteur de libération de l'hormone de croissance humaine (HGRF), une hormone lutéinisante (LH) ou un facteur de croissance épidermique.

16. Forme galénique selon la revendication 14 ou 15, **caractérisée en ce que** la couche de matrice est un acide gras en $C_6$ à $C_{20}$ et/ou un alcool en $C_6$ à $C_{20}$, y compris leurs sels, dérivés d'éther, ester ou amide, et/ou un lipide et/ou un phospholipide et/ou une vitamine liposoluble et/ou un inhibiteur de la protéase et/ou un agent de renforcement de la pénétration.

17. Forme galénique selon une ou plusieurs des revendications 1 à 16, **caractérisée en ce que** entre la couche de matrice contenant le principe actif et la couche de revêtement pelliculé externe est appliquée une couche de séparation.

18. Procédé de fabrication d'un médicament multiparticulaire selon une ou plusieurs des revendications 1 à 17, dans lequel :

   a) une couche de matrice interne contenant un principe actif qui est un peptide ou une protéine et un polymère à effet muco-adhésif, et éventuellement d'autres adjuvants pharmaceutiques habituels est produite par application par pulvérisation sur un noyau ou par agglomération rotative, précipitation ou par un procédé de pulvérisation sans noyau, et ensuite
   b) un revêtement pelliculé externe constitué essentiellement d'un polymère ou copolymère anionique qui peut être formulé éventuellement avec des adjuvants pharmaceutiques habituels, en particulier des plastifiants, est appliqué par application par pulvérisation de sorte que des granulés enrobés contenant le principe actif sont obtenus, et
   c) on transforme les granulés obtenus au moyen d'adjuvants pharmaceutiques habituels de manière connue en soi en une forme galénique multiparticulaire, en particulier en comprimés, mini-comprimés, capsules, sachets ou sirops secs contenant les granulés qui sont formulés de telle sorte que les granulés contenus soient libérés dans la plage de pH de l'estomac.

19. Forme galénique selon une ou plusieurs des revendications 1 à 17, **caractérisée en ce que** le principe actif est intégré dans une matrice lipophile qui présente un point de fusion supérieur à 37° C et la matrice lipophile contenant le principe actif est intégrée dans la matrice constituée du polymère à effet muco-adhésif.

20. Forme galénique selon la revendication 19, **caractérisée en ce que** le principe actif et la ou les substances qui forment la matrice lipophile se distinguent par leur solubilité dans l'eau selon le DAB 10 inférieure ou égale à +/- 50 %, et/ou se distinguent par leur coefficient de distribution selon l'annexe V du RL67/548/CEE A.8, inférieur ou égal à +/-.60 % et/ou par leur valeur HLB mesurée selon Marszall, inférieure ou égale à +/- 80 %

21. Forme galénique selon la revendication 19 ou 20, **caractérisée en ce qu'**elle contient un principe actif, qui présente une solubilité dans l'eau selon le DAB 10 d'au moins 30 parties en volume d'eau pour une proportion en poids de principe actif.

22. Forme galénique selon la revendication 21, **caractérisée en ce que** le principe actif est choisi dans le groupe comprenant les antibiotiques peptidiques, les immunosuppresseurs, les antagonistes de LHRH, les immunomodulateurs.

23. Forme galénique selon la revendication 21 ou 22, **caractérisée en ce que** le principe actif est l'abarelix, l'angiotensine II, l'anidulafungine, l'antide, l'argipressine, l'azaline et l'azaline B, l'antagoniste de la bombésine, la bradykinine, la buséréline, la calcitonine, le cetrorelix, la ciclosporine, la ciclosporine A, la desmopressine, le detirelix, l'érythropoïétine, l'encéphaline (Leu-, Met-), le ganirelix, la gonadoreline, la gosereline, l'hormone de croissance sécrétagogue, l'insuline, l'interféron (alpha, bêta, gamma), l'interleukine (IL1, IL2), la micafungine, la nafaréline, le leuprolide, la leuproréline, l'octréotide, l'orntide, l'oxytocine, l'hormone parathyroïdienne, le ramorelix, la sécrétine, la somatotropine, la terlipressine, le tétracosactide, le teverelix, la triptoréline, la thyrolibérine, la thyrotropine, le facteur de nécrose tumorale (TNF alpha, bêta) ou la vasopressine.

24. Forme galénique selon une ou plusieurs des revendications 19 à 23, **caractérisée en ce que** la ou les substances qui forment la matrice lipophile et le polymère à effet muco-adhésif présentent soit la même propriété ionique, soit, dans le cas de propriétés ioniques opposées, le polymère à effet muco-adhésif se présente au moins à 50 % sous forme neutralisée.

25. Forme galénique selon une ou plusieurs des revendications 19 à 24, **caractérisée en ce que** la matrice lipophile est constituée de 80 à 100 % en poids d'une substance présentant une valeur HLB de 0 à 15 ou d'un mélange de substances présentant une valeur HLB moyenne de 0 à 15 et peut contenir de 0 à 20 % en poids d'adjuvants pharmaceutiques habituels, en particulier de stabilisants, d'agents épaississants ou d'absorbants.

26. Forme galénique selon une ou plusieurs des revendications 19 à 25, **caractérisée en ce que** la ou les substances qui forment la matrice lipophile font partie du groupe des huiles, graisses, mono-, di- ou triglycérides, acides gras, alcools gras, en particulier un acide gras en $C_6$ à $C_{20}$ et/ou un alcool en $C_6$ à $C_{20}$, y compris leurs sels, dérivés d'éther, ester ou amide, et/ou un phospholipide, une lécithine, des émulsifiants, des lipoïdes, des vitamines liposolubles ou des tensioactifs.

27. Forme galénique selon une ou plusieurs des revendications 19 à 25, **caractérisée en ce que** la matrice lipophile contient l'une des préparations lipidiques suivantes : (Imwitor 308) glycéryl-monocaprylate présentant une teneur en mono-ester > 80 %, (Imwitor 312) glycéryl-monolaurate présentant une teneur en mono-ester > 90 %, (Imwitor 491) Glycérolmonostéarate ($C_{16}$ + $C_{18}$) présentant une teneur en mono-ester > 90%, (Imwitor 900 P) glycérol-monostéarate présentant une teneur en mono-ester de 40 à 55 % et une teneur en $C_{18}$ de 40 à 60 %, (Imwitor 900 K) glycérolmonostéarate, présentant une teneur en mono-ester de 40 à 55 % et une teneur en $C_{18}$ de 60 à 80 %, (Imwitor 742) glycéride à chaîne moyenne en $C_8$ et $C_{10}$ présentant une teneur en mono-ester de 45 à 55 %, (Imwitor 928) glycérides partiels d'acides gras végétaux saturés en $C_{10}$ à $C_{18}$ présentant une teneur principale en $C_{12}$ et une teneur en mono-ester de 34 à 36 %, des glycérides en $C_8$ et $C_{10}$, du caprylate de sodium ou du capriate de sodium.

28. Forme galénique selon une ou plusieurs des revendications 19 à 27, **caractérisée en ce que** le principe actif est soluble à au moins 10 % dans la matrice lipophile.

29. Forme galénique selon une ou plusieurs des revendications 19 à 28, **caractérisée en ce que** la teneur en matrice lipophile contenant le principe actif sur la couche de matrice interne a) est de 5 à 50 % en poids.

30. Procédé de fabrication d'une forme galénique multiparticulaire selon une ou plusieurs des revendications 19 à 29, comprenant les étapes consistant à :

   a) produire la matrice lipophile contenant le principe actif par mise en suspension et/ou dissolution du principe actif avec la ou les substances qui forment la matrice lipophile et éventuellement d'autres adjuvants pharmaceutiques habituels par mélange intensif ou fusion des composants,
   b) produire des pré-granulés (noyaux de granulés) par application par pulvérisation du polymère muco-adhésif en mélange avec la matrice lipophile contenant le principe actif sur un noyau ou par agglomération rotative, précipitation ou procédé par pulvérisation sans noyau,

c) produire des granulés par application par pulvérisation d'un revêtement du polymère ou copolymère anionique qui peut contenir des charges d'adjuvants pharmaceutiques habituels, en particulier des plastifiants et des agents de séparation, d'une dispersion ou d'une solution organique sur les pré-granulés de l'étape b),

d) fabriquer une forme galénique multiparticulaire par garnissage ou enrobage des granulés de l'étape c) de façon connue en soi, éventuellement en utilisant des adjuvants pharmaceutiques habituels, en particulier par transformation en comprimés, mini-comprimés, capsules, sachets ou sirops secs contenant les granulés.

**31.** Procédé de fabrication d'une forme galénique multiparticulaire selon la revendication 30, **caractérisé en ce que** les étapes a) et b) sont réalisées comme suit :

a) production de la couche de matrice interne par fabrication d'une émulsion, dispersion ou solution de principe actif avec la ou les substances pour la matrice lipophile, et éventuellement d'autres adjuvants pharmaceutiques habituels par mélange intensif des composants dans l'eau et production d'une préparation huile dans eau présentant une taille de particules moyenne qui ne dépasse pas 60 $\mu$m,

b) production de pré-granulés par application par pulvérisation de la préparation huile dans eau de l'étape a) sur le polymère muco-adhésif qui peut contenir éventuellement des charges d'autres adjuvants pharmaceutiques habituels, les composants se présentant sous la forme d'une poudre micronisée, par agglomération rotative, extrusion ou granulation.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 10024451 A1
- WO 0203955 A
- WO 0264148 A
- WO 0243767 A
- WO 03007913 A
- WO 9313753 A
- EP 0704207 A2
- EP 0704208 A2
- DE 2135073 C
- EP 0262326 A
- EP 0683028 A
- WO 9601624 A
- US 4795643 A

### In der Beschreibung aufgeführte Nicht-Patentliteratur

- **HASSAN E.E. ; GALLO J. M.** A Simple Rheological Method for the in Vitro Assessment of Mucin-Polymer Bioadhaesive Bond Strength. *Pharma Res.,* 1990, vol. 7 (5), 491
- **RUDOLF VOIGT.** Lehrbuch der pharmazeutischen Technologie. Verlag Chemie, 1984, vol. 5, 151
- **BAUER ; LEHMANN ; OSTERWALD ; ROTH-GANG.** Überzogene Arzneiformen. *Wissenschaftliche Verlagsgesellschaft mbH,* 165-196
- **VOIGT, R.** Lehrbuch der pharmazeutischen Technologie. Verlag Chemie, 1984
- **SUCKER, H. ; FUCHS, P. ; SPEISER, P.** Pharmazeutische Technologie. Georg Thieme Verlag, 1991, 626-642
- Remington's Pharmaceutical Sciences. Mack Publishing Co, 1985, 1567-1573
- **LIST, P. H.** Arzneiformenlehre, Wissenschaftliche Verlagsgesellschaft mbH. 1982
- Deutsche Forschungsgemeinschaft. Farbstoffe für Lebensmittel. Harald Boldt Verlag, 1978
- *Deutsche Lebensmittelrundschau,* 1978, vol. 74 (4), 156
- Farbstoffe für Lebensmittel. *Deutsche Forschungsgemeinschaft,* 1978
- **BECKERT et al.** Compression of enteric-coated pellets to disintegrating tablets. *International Journal of Pharmaceutics,* 1996, vol. 143, 13-23
- *Parfümerie, Kosmetik,* 1979, vol. 60, 444-448